# FASCICULE DE BREVET EUROPEEN

(11) **EP 0 356 271 B1**
(45) Date de publication et mention de la délivrance du brevet: **05.04.1995**
(21) Numéro de dépôt: 89402002.3
(22) Date de dépôt: 12.07.1989
(51) Int. Cl.: C07D 239/48, A61K 7/06

(54) **Nouveaux dérivés de diamino-2,4 pyrimidine oxyde-3 et leur utilisation pour le traitement et la prévention de la chute des cheveux**
2,4-Diamino-pyrimidin-3-oxid-Derivate und deren Verwendung bei der Behandlung und zur Verhütung von Haarfall
2,4-Diamino-pyrimidine-3-oxide derivatives and their use in the treatment and the prevention of hair drop

(30) Priorité: 01.08.1988 LU 87308
(43) Date de publication de la demande: 28.02.1990
(73) Titulaire: L'OREAL, 75008 Paris (FR)
(72) Inventeur: Hocquaux, Michel, F-75012 Paris (FR); Dumats, Jacqueline, F-93420 Villepinte (FR); Gaetani, Quintino, F-93270 Sevran (FR)
(74) Mandataire: Casalonga, Axel

(56) Documents cités:
- WO-A-86/00616

## Description

La présente invention est relative à ce nouveaux dérivé de pyrimidine oxyde-3, à leur préparation et à des compositions cosmétiques ou pharmaceutiques destinées notamment à être utilisées en application topique dans le traitement et la prévention de la chute des chevaux.

On connaît déjà, dans l'état de la technique, le pipéridine-6 diamino-2,4 pyrimidine oxyde-3 ou "Minoxidil" pour ses propriétés d'agent antihypertenseur, mais également pour son utilisation dans le traitement de la chute des cheveux, de la pelade, de la dermatite desquamante, de l'alopécie, etc.

D'autres dérivés de la diamino-2,4 pyrimidine oxyde-3 substitués en position 6 par une amine, ont été décrits dans la demande WO-A-8600616 comme agents pour la croissance des cheveux.

La demanderesse vient de découvrir de nouveaux produits, dérivés de la pyrimidine oxyde-3, substitués en position 6.

Elle a découvert que ces produits étaient particulièrement efficaces pour la repousse des cheveux, en particulier pour induire et stimuler la croissance des cheveux et freiner leur chute et pouvaient être utilisés, notamment, dans le traitement des maladies du cuir chevelu, telles que la pelade, la dermatite desquamante, l'alopécie.

Ces composés présentent, par ailleurs, une activité antihypertensive sensiblement plus faible que celle du Minoxidil.

L'invention a donc pour objet de nouveaux dérivés de pyrimidine oxyde-3 substitués en position 6.

Un autre objet de l'invention est constitué par leur procédé de préparation.

L'invention concerne également des compositions cosmétiques et/ou pharmaceutiques mettant en oeuvre ces composés.

D'autres objets de l'invention apparaîtront à la lecture de la description et des exemples qui suivent.

Les composés conformes à l'invention sont essentiellement caractérisés par le fait qu'ils répondent à la formule : 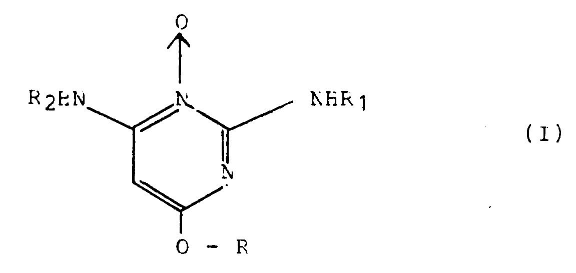
dans laquelle :
R₁ et R₂ désignent, indépendamment l'un de l'autre, un atome d'hydrogène, un groupement carbamoyle de formule : 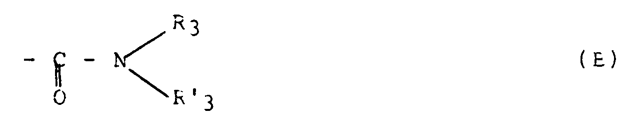
avec R'₃ = H ou R₃;
ou un groupement acyle de formule : 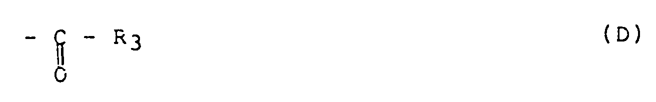
dans lesquels R₃ désigne un radical alkyle linéaire ou ramifié en C₁-C₁₈, un groupement alcényle en C₂-C₁₈, un groupement cycloalkyle en C₅-C₈; R₃ peut également désigner un radical aryle ou aralkyle répondant à la formule : 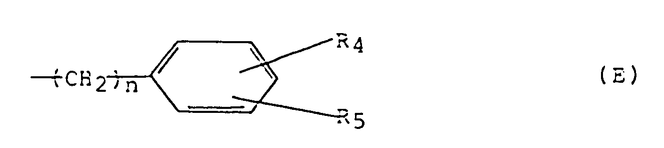
dans laquelle :
n est un nombre entier pouvant varier entre 0 et 4;
R₄ et/ou R₅, indépendamment l'un de l'autre, désignant hydrogène, un groupement alkyle inférieur en C₁-C₆, un groupement hydroxyle, alcoxy en C₁-C₆, un atome d'halogène ou un groupement CF₃;
R désigne un radical alkyle, linéaire ou ramifié en C₁-C₁₈,
alcényle en C₂-C₁₈, un radical cycloalkyle en C₄-C₆ pouvant porter une insaturation ou un radical alkyle en C₁-C₆ portant un groupement phényle ou phényle ou pyridine.

Les composés plus particulièrement préférés, conformes à l'invention, sont les composés dans lesquels le groupement alkyle désigne, à défaut d'indication contraire, un groupement ayant 2 à 12 atomes de carbone, le noyau aromatique désigne de préférence phényle.

Les composés particulièrement préférés, définis ci-dessus, sont ceux dans lesquels R est choisi parmi les groupements méthyle, éthyle, butyle, isobutyle, n-hexyle, n-octyle, n-décyle, lauryle, n-héxényl-5, éthyl-2 hexyle, undécényl-10, cyclohexyle, phénéthyle ou benzyle.

Les composés particulièrement préférés de l'invention sont constitués par le diamino-2,4 (n-butyloxy)-6 pyrimidine oxyde-3, le diamino-2,4 éthyloxy-6 pyrimidine oxyde-3, le diamino-2,4 méthoxy-6 pyrimidine oxyde-3, le diamino-2,4 n-hexyloxy-6 pyrimidine oxyde-3, le diamino-2,4 n-octyloxy-6 pyrimidine oxyde-3, le diamino-2,4 n-dodécyloxy-6 pyrimidine oxyde-3, le diamino-2,4 (éthyl-2 hexyloxy)-6 pyrimidine oxyde-3, le diamino-2,4 (n-héxényl-5 oxy)-6 pyrimidine oxyde-3, le diamino-2,4 (undécényl-10 oxy)-6 pyrimidine oxyde-3, le diamino-2,4 (phényl-2 éthyloxy)-6 pyrimidine oxyde-3, le diamino-2,4 (trifluoro-2 éthyloxy)-6 pyrimidine oxyde-3, l'amino-2 acétamido-4 butyloxy-6 pyrimidine oxyde-3, le diacétamido-2,4 butyloxy-6 pyrimidine oxyde-3, le di(méthoxycarbonyl amino)-2,4 butyloxy-6 pyrimidine oxyde-3, le di(benzyl oxycarbonylamino)-2,4 butyloxy-6 pyrimidine oxyde-3, la N(amino-2 oxyde-3 butyloxy-6 pyrimidinyl-4)N′-diméthyl urée.

Les composés conformes à l'invention peuvent également exister sous leur forme tautomère, répondant aux formules (IA) et (IB) suivantes : 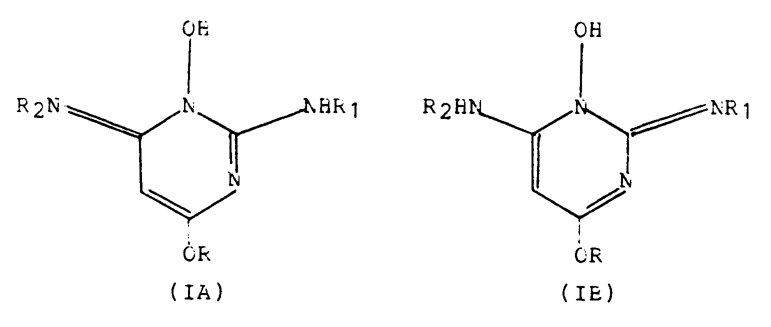
Ces formes tautomères (I), (IA) et (IB) peuvent être présentes dans des proportions variables et l'une peut être prépondérante par rapport aux autres.

Les composés conformes à l'invention pour lesquels R₁ et R₂ désignent hydrogène sont préparés en partant du diamino-2,4(dichloro-2,4 phénoxy)-6 pyrimidine oxyde-3 ou du diamino-2,4 chloro-6 pyrimidine oxyde-3 que l'on fait réagir avec un alcoolate de formule RO^{⊖}Y^{⊕} ou R a la spécification indiquée ci-dessus et Y est un cation alcalin tel que le sodium, le potassium, le lithium.

Les composés pour lesquels R₁ et R₂ sont des groupements carbamoyle, alcoxycarbonyle ou acyle, sont obtenus à partir des dérivés diamino-2,4 alcoxy-6 pyrimidine oxyde-3 correspondants dont la préparation est décrite ci-après.

L'obtention de dérivés pour lesquels R₁ et R₂ sont des groupements carbamoyle, est généralement réalisée en faisant réagir un chlorure de carbamoyle sur le dérivé diamino-2,4 alcoxy-6 pyrimidine oxyde-3 correspondant dans un solvant polaire, tel que le diméthylsulfoxyde, à un température comprise entre 0 et 100°C et plus particulièrement entre 20 et 70°C.

L'obtention de dérivés pour lesquels R₁ et R₂ sont des groupements alcoxycarbonyle, est généralement réalisée par l'action d'un excès d'ester chloroformique sur le dérivé diamino-2,4 alcoxy-6 pyrimidine oxyde-3 correspondant en opérant dans un solvant polaire aprotique, tel que le dichlorométhane en présence d'une amine tertiaire, telle que la triéthylamine ou la pyridine, à une température comprise entre 0 et 50°C.

L'obtention de dérivés pour lesquels R₁ et R₂ sont des groupements acyle, est généralement réalisée en faisant réagir un chlorure d'acide ou un anhydride sur le dérivé diamino-2,4 alcoxy-6 pyrimidine 3-oxyde correspondant dans un solvant polaire aprotique comme le dichlorométhane, en présence d'une amine tertiaire telle que la triéthylamine ou la pyridine, à une température comprise entre 0 et 50°C.

Un autre objet de l'invention est constitué par l'utilisation de certains dérivés de diamino-2,4 pyrimidine oxyde-3 en tant qu'intermédiaires pour la préparation de dérivés d'oxadiazolopyrimidines par une réaction de cyclisation-élimination.

En effet, en partant des dérivés de pyrimidine mono- ou dicarbamate (où R₁ et R₂ ont la signification (C) définie précédemment), ou bien de pyrimidine mono-ou diuréido (où R₁ et R₂ ont la signification (B) définie ci-dessus), on peut obtenir des oxadiazolo-pyrimidines suivant le schéma réactionnel suivant : 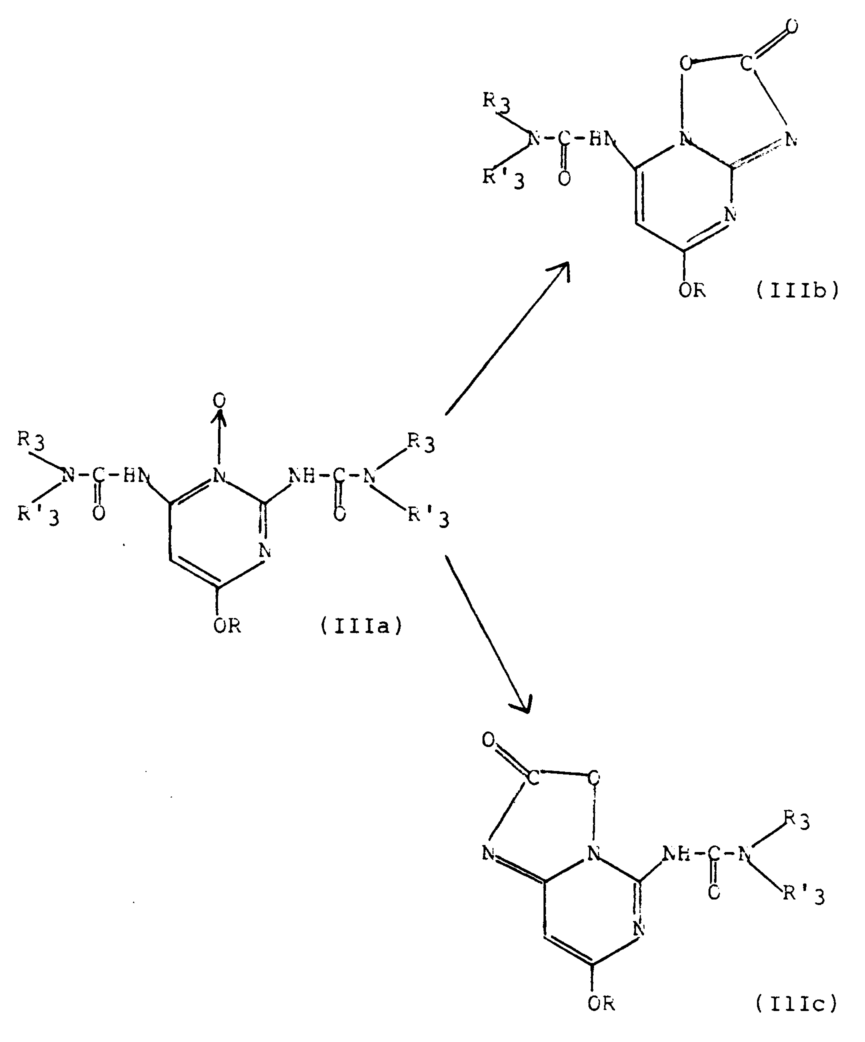 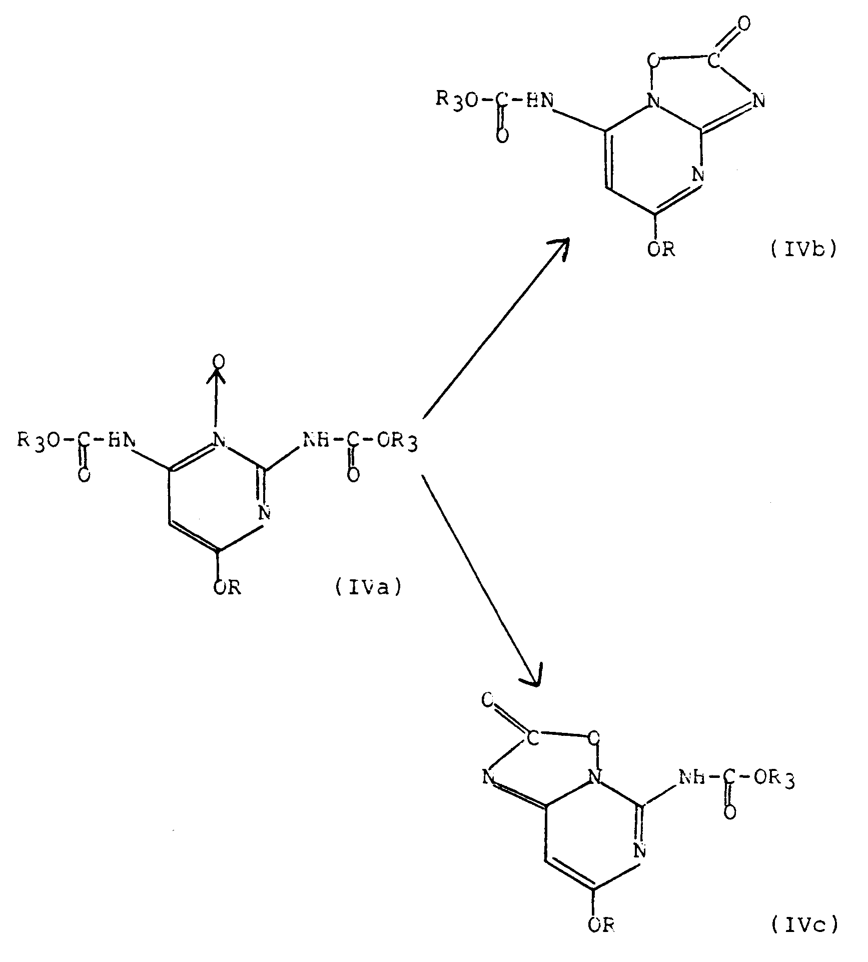
où R, R₃, et R′₃ ont les significations définies précédemment.

La diurée (IIa) est portée, pendant quelques heures, à une température comprise entre 50 et 120°C dans un solvant organique, tel que le toluène et conduit à l'oxadiazolopyrimidine de structure (IIIb).

En cyclisant, par exemple, un composé de formule (IVa) ci-dessus, on obtient finalement une oxadiazolopyrimidine de formule (IVb). Cette réaction connue est réalisée en chauffant un composé de formule (IVa) à une température d'environ 50 à 200°C, de préférence d'environ 100 à 150°C. La réaction peut être effectuée en l'absence ou en présence d'un solvant ou d'un mélange de solvants. Si la réaction est effectuée dans un solvant ou un mélange de solvants, on pourra utiliser en particulier des hydrocarbures aromatiques comme le benzène, le toluène ou le xylène, des hydrocarbures chlorés comme le chloroforme, des alcools comme le butanol ou l'isobutanol, des éthers comme l'éther butylique, le dioxanne ou l'éther diméthylique du diéthylèneglycol, le diméthylformamide, le diméthylsulfoxyde, et les solvants analogues ou leurs mélanges.

Les oxadiazolopyrimidines de formules (IIIb) et (IVb) peuvent être converties de manière connue en soi, en sels acceptables pour l'usage pharmaceutique à l'aide de bases minérales ou organiques. 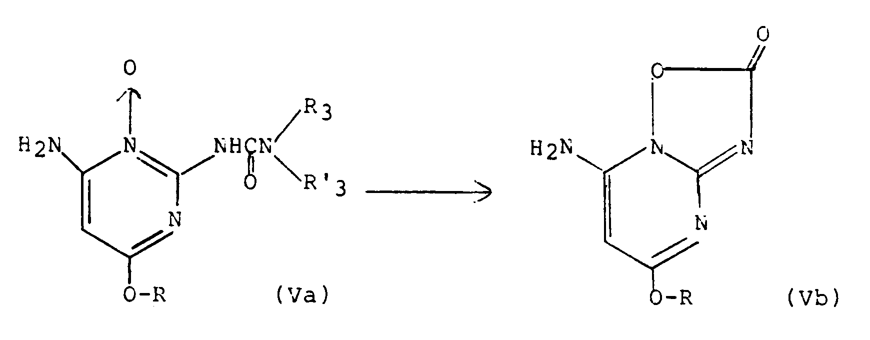
A partir de l'urée de formule (Va), on obtient, d'une manière quantitative, le composé de formule (Vb) conforme à l'invention, en portant l'urée (Va) à une température comprise entre 40 et 100°C, pendant quelques heures, dans un solvant organique, tel que le toluène. 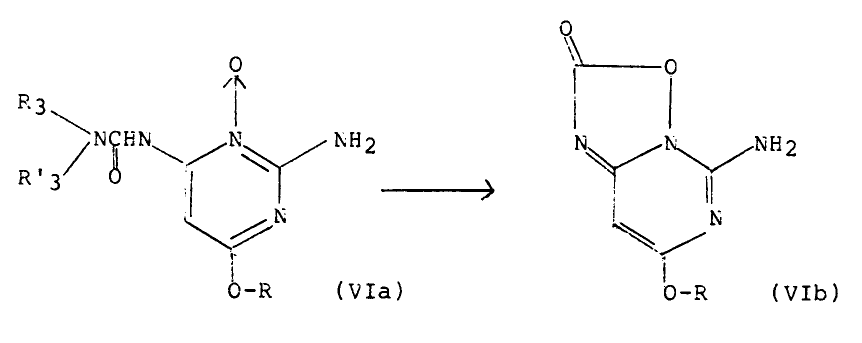
La forme isomère de structure (VIb), conforme à l'invention, est obtenue en portant l'urée de formule (VIa) dans un solvant organique, tel que le toluène ou le xylène, à une température supérieure à 100°C, pendant quelques heures.

A partir des composés de formule (I), on peut préparer leurs sels d'addition d'acides cosmétiquement ou pharmaceutiquement acceptables, tels que les sels des acides sulfurique, chlorohydrique, bromhydrique, phosphorique, acétique, benzoïque, salicylique, glycolique, succinique, nicotinique, tartrique, maléïque, pamoïque, méthane sulfonique, picrique, lactique, etc.

Les composés conformes à l'invention peuvent être utilisés dans le domaine cosmétique ou pharmaceutique, notamment dans les applications topiques, et plus particulièrement dans le traitement ou la prévention de la chute des cheveux, et plus particulièrement de la pelade, de l'alopécie, ainsi que des dermatites desquamantes.

Ces compositions sont essentiellement caractérisées par le fait qu'elles contiennent dans un milieu physiologiquement acceptable, approprié pour une application topique, au moins un composé répondant à la formule (I) ou un de ses sels.

Ces compositions peuvent comporter à titre de milieu physiologiquement acceptable, tout milieu approprié pour l'application topique, soit en cosmétique, soit en pharmacie, et qui soit compatible avec la substance active.

Les composés conformes à l'invention peuvent se trouver dans ce milieu, soit à l'état dissous, soit à l'état dispersé, notamment sous forme micronisée.

Les compositions destinées à être utilisées en pharmacie se présentent sous forme d'onguent, de teinture, de crème, de pommade, de poudre, de timbre, de tampon imbibé, de solution, d'émulsion ou d'émulsion vésiculaire, de lotion, de gel, de spray ou de suspension. Elles peuvent être, soit anhydres, soit aqueuses, selon l'indication clinique.

Les composés sont présents dans ces compositions pharmaceutiques à des concentrations comprises entre 0,1 et 10% en poids, et en particulier comprises entre 0,2 et 5% en poids.

Les compositions cosmétiques sont notamment destinées à être utilisées sous forme de lotion, de gel, de savon, de shampooing, d'aérosol ou de mousse et contiennent, dans un support physiologiquement acceptable, au moins un composé de formule (I) ou l'un de ses sels.

La concentration des composés de formule (I) dans ces compositions est, de préférence, comprise entre 0,01 et 7,5% en poids et en particulier entre 0,05 et 5% en poids.

Les compositions conformes à l'invention peuvent contenir différents additifs habituellement utilisés en cosmétique ou en pharmacie et en particulier des substances actives, telles que des agents hydratants comme la thiamorpholine et ses dérivés ou l'urée; des agents antiséborrhéiques tels que la S-carboxyméthylcystéine, la S-benzylcystéamine et leurs dérivés; la thioxolone.

Les composés conformes à l'invention peuvent être associés à les composés améliorant encore leur activité sur la repousse et/ou sur la freinage de la chute des cheveux, tels que plus particulièrement les composés suivants :
- les esters d'acide nicotinique, dont plus particulièrement les nicotinates d'alkyle en C₁-C₆ et notamment le nicotinate de méthyle;
- les agents anti-inflammatoires stéroïdiens et non stéroïdiens bien connus dans l'état de la technique et en particulier l'hydrocortisone, ses sels et ses dérivés, l'acide niflumique, etc;
- les rétinoïdes et plus particulièrement l'acide t-trans rétinoïque appelé encore trétinoïne, l'isotrétinoïne, le rétinol ou vitamine A et ses dérivés, tels que l'acétate, le palmitate ou le propionate, le motrétinide, l'étrétinate, le t-trans rétinoate de zinc;
- les agents antibactériens choisis plus particulièrement parmi les macrolides, les pyranosides et les tétracyclines et notamment l'érythromycine;
- les agents antagonistes de calcium, tels que plus particulièrement la cinnarizine et le diltiazem;
- des hormones, telles que l'estriol ou des analogues ou la thyroxine et ses sels;
- des agents antiandrogènes, tels que l'oxendolone, la spironolactone, le diéthylstilbestrol;
- des capteurs de radicaux OH, tels que le diméthylsulfoxyde.

On peut également associer avec les composés de l'invention, éventuellement en mélange avec les autres, des composés tels que le diazoxyde correspondant au méthyl-3 chloro-7 2H benzothiadiazine 1,2,4-dioxyde-1,1; la spiroxasone ou 7-(acétylthio)-4′,5′-dihydrospiro [androst 4-ène-17,2′-(3′H)furan]-3 one; des phospholipides, tels que la lécithine; les acides linoléique et linolénique; l'acide salicylique et ses dérivés décrits dans le brevet français 2 581 542, et plus particulièrement les dérivés d'acide salicylique porteurs d'un groupement alcanoyle ayant 2 à 12 atomes de carbone en position 5 du cycle benzénique; des acides hydroxycarboxyliques ou cétocarboxyliques et leurs esters, des lactones et leurs sels correspondants; l'anthraline ou le trihydroxy-1,8,9 anthracène, les caroténoîdes, acides eicosatetraynoîque et eicosatriynoîque, leurs esters et amides.

Les composés conformes à l'invention peuvent également être associés à des agents tensio-actifs dont plus particulièrement ceux choisis parmi les agents tensio-actifs non ioniques et amphotères.

Parmi les tensio-actifs non ioniques, on citera notamment les polyhydroxypropyléthers décrits dans les brevets français n° 1 477 048; 2 091 516; 2 169 787; 2 328 763; 2 574 786; les alkyl(C₈-C₉) phénols oxyéthylénés comportant de 1 à 100 moles d'oxyde d'éthylène et de préférence 5 à 35 moles d'oxyde d'éthylène; les alkylpolyglycosides de formule :

CₙH₂ₙ₊₁(C₆H₁₀O₅)ₓH (A)

dans laquelle n varie de 8 à 15 inclus et x de 1 à 10 inclus.

Parmi les agents tensio-actifs amphotères, on citera plus particulièrement les amphocarboxylglycinates et les amphocarboxypropionates définis dans le dictionnaire CTFA, 3ème édition, 1982, et vendus, notamment, sous la dénomination MIRANOL® par la Société MIRANOL.

Les composés, selon l'invention, peuvent être introduits dans des supports qui améliorent encore l'activité au niveau de la repousse, en présentant à la fois des propriétés avantageuses sur le plan cosmétique, telles que des mélanges volatils ternaires d'alkyléther d'alkylèneglycol ou de dialkylèneglycol (alkyle et alkylène de préférence en C₁ à C₄), d'alcool éthylique et d'eau, le solvant glycolique désignant plus particulièrement le monoéthyléther de l'éthylène glycol, le monométhyléther du propylèneglycol, le monoéthyléther du diéthylèneglycol.

Les composés conformes à l'invention peuvent également être introduits dans des supports gélifiés ou épaissis, tels que des supports essentiellement aqueux gélifiés par des hétérobiopolysaccharides, tels que la gomme de xanthane ou les dérivés de cellulose, des supports hydroalcooliques gélifiés par des polyhydroxy éthylacrylate ou méthacrylate ou des supports essentiellement aqueux épaissis, en particulier par des acides polyacryliques réticulés par un agent polyfonctionnel, tel que les Carbopol vendus par la Société GOODRICH.

Ces compositions peuvent également contenir des agents conservateurs, des agents stabilisants, des agents régulateurs de pH, des agents modificateurs de pression osmotique, des agents émulsifiants, des filtres UVA et UVB, des agents antioxydants tels que l' α -tocophérol, le butylhydroxyanisole, le butylhydroxytoluène.

Le milieu physiologiquement acceptable peut être constitué par de l'eau ou un mélange d'eau et d'un solvant ou un mélange de solvants, les solvants étant choisis parmi les solvants organiques acceptables sur le plan cosmétique ou pharmaceutique et choisis plus particulièrement parmi les alcools inférieurs en C₁-C₄, comme l'alcool éthylique, l'alcool isopropylique, l'alcool tertiobutylique, les alkylèneglycols, les alkyléthers d'alkylèneglycol et de dialkylèneglycol, tels que le monoéthyléther d'éthylèneglycol, le monométhyléther de propylèneglycol, le monoéthyléther de diéthylèneglycol. Les solvants, lorsqu'ils sont présents, le sont dans des proportions comprises entre 1 et 80% en poids par rapport au poids total de la composition.

Les milieux physiologiquement acceptables peuvent être épaissis à l'aide d'agents épaississants habituellement utilisés en cosmétique ou pharmacie, et on peut plus particulièrement citer les hétérobiopolysaccharides tels que la gomme de xanthane, les scléroglucanes, les dérivés de cellulose comme les éthers de cellulose, les polymères acryliques, réticulés ou non.

Les épaississants sont présents de préférence dans des proportions comprises entre 0,1 et 5% en poids et en particulier entre 0,4 et 3% en poids par rapport au poids total de la composition.

L'invention a également pour objet un procédé de traitement cosmétique des cheveux ou du cuir chevelu, consistant à leur appliquer au moins une composition telle que définie ci-dessus, en vue d'améliorer l'esthétique de la chevelure.

Un autre objet de l'invention est constitué par l'utilisation de la composition définie ci-dessus, pour la préparation d'un médicament ayant pour effet d'induire ou de stimuler la croissance des cheveux et de freiner leur chute.

Le traitement consiste principalement à appliquer sur les zones alopéciques du cuir chevelu d'un individu, la composition telle que définie ci-dessus.

Le mode d'application préféré consiste à appliquer 1 à 2 g de la composition sur la zone alopécique, à une fréquence de une à deux applications par jour, pendant 1 à 7 jours par semaine et ceci pendant une durée de 1 à 6 mois.

Les compositions peuvent notamment être utilisées dans le traitement de la pelade, de la chute des cheveux, de la dermatite desquamante.

Les exemples suivants sont destinés à illustrer l'invention.

### EXEMPLE 1

### Diamino-2,4 (n-butyloxy)-6 pyrimidine oxyde-3

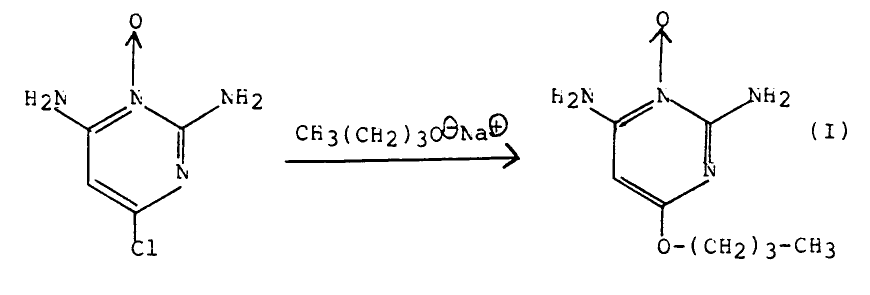

Le composé (I) est obtenu par réaction du n-butylate de sodium sur le diamino-2,4 chloro-6 pyrimidine oxyde-3.

### Mode opératoire

4 g de sodium sont dissous à 80°C dans 150 ml de n-butanol séché. Après addition de 20 g de diamino-2,4 chloro-6 pyrimidine oxyde-3, le mélange réactionnel est maintenu 24 heures à reflux.

Après addition de 500 cm³ de dichlorométhane, l'insoluble formé est filtré. On récupère la phase organique, on évapore et on reprend la substance dans 500 cm³ de CH₂Cl₂, on lave par une solution aqueuse saturée en NaCl, on sèche sur Na₂SO₄ et on évapore à sec.

Le précipité blanc obtenu est repris dans du CH₂Cl₂ et purifié par précipitation dans un mélange méthanol/hexane.

On obtient 3,5 g du composé (I).
Rendement : 14%.
F = 166°C.

| Analyse élémentaire : C₈H₁₄N₄O₂ ; PM = 198. | | | | |
|---|---|---|---|---|
| | C | H | N | O |
| Calculé | 48,48 | 7,07 | 28,28 | 16,16 |
| Trouvé | 47,87 | 7,11 | 27,97 | 16,24 |

Les spectres ¹H RMN et de masse sont conformes à la structure attendue.

### Résultats pharmacologiques

Outre son activité anti-chute et stimulatrice de la repousse des cheveux, le composé (I) présente un faible effet anti-hypertenseur sur des rats spontanément hypertendus.

### EXEMPLE 2

### Diamino-2,4 éthyloxy-6 pyrimidine oxyde-3

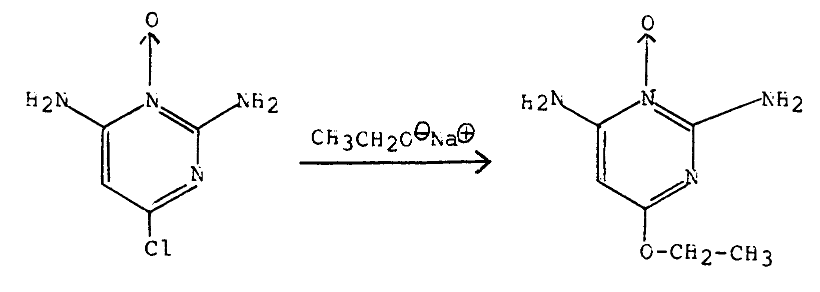

On dissout 1,8 g de sodium dans 100 ml d'éthanol. On ajoute 10 g de diamino-2,4 chloro-6 pyrimidine oxyde-3. On porte le milieu réactionnel au reflux jusqu'à disparition du produit de départ et l'on chauffe à 120°C dans un autoclave pendant 7 heures. On revient à la température ambiante et on filtre l'insoluble et lave par l'éthanol.

On concentre la phase alcoolique. On ajoute de l'éthanol chlorohydrique jusqu'à pH acide. On agite puis on précipite le chlorohydrate par de l'éther anhydre. On filtre et on sèche.

On reprend le précipité blanc dans H₂O (1 g dans 10 cm³). On amène à pH basique. On refroidit, on filtre le précipité. On lave par l'acétone, puis l'éther anhydre.

| Analyse élémentaire : C₆H₁₀N₄O₂; PM = 170. | | | | |
|---|---|---|---|---|
| | C | H | O | N |
| Calculé | 42,35 | 5,88 | 18,52 | 32,94 |
| Trouvé | 42,41 | 6,04 | 19,03 | 32,69 |

Les spectres RMN ¹H et ¹³C et de masse sont conformes à la structure attendue.

Point de fusion : décomposition vers 252°C.

### EXEMPLE 3

### Diamino-2,4 méthoxy-6 pyrimidine oxyde-3

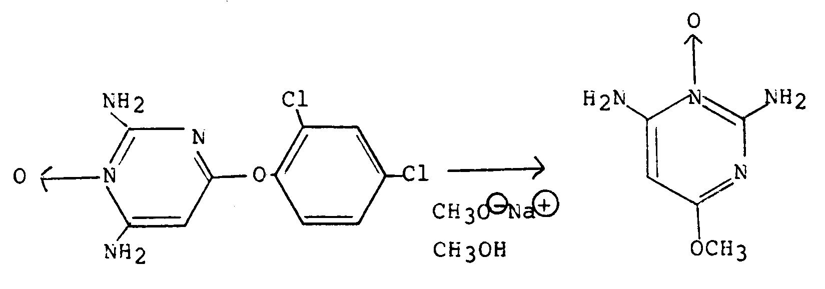

On dissout 1,8 g de sodium dans 150 ml de méthanol. On ajoute 15 g de diamino-2,4 (dichloro-2,4 phénoxy)-6 pyrimidine oxyde-3 et on porte le milieu réactionnel à reflux pendant 15 jours.

On évapore le solvant et le résidu obtenu est chromatographie sur gel de silice (éluant : acétate d'éthyle 85/méthanol 15).

Le précipité blanc obtenu est recristallisé dans un mélange acétonitrile/méthanol.
Rendement = 20%
F = décomposition à 232°C.

| Analyse élémentaire : C₅H₈N₄O₂; M = 156. | | | | |
|---|---|---|---|---|
| | C | H | N | O |
| Calculé | 38,46 | 5,13 | 35,90 | 20,51 |
| Trouvé | 38,28 | 5,18 | 35,87 | 20,53 |

Les spectres ¹H RMN et de masse sont conformes à la structure attendue.

### EXEMPLE 4

### Diamino-2,4 n-hexyloxy 6-pyrimidine oxyde-3

La préparation est effectuée suivant le mode opératoire décrit à l'exemple 3 avec le n-hexanol.
Température : 120°C Temps : 20 heures
Recristallisation dans un mélange méthanol/éther
Rendement : 10%
F = 184°C.

| Analyse élémentaire : C₁₀H₁₈N₄O₂; M = 226. | | | | |
|---|---|---|---|---|
| | C | H | N | O |
| Calculé | 53,10 | 7,96 | 24,78 | 14,16 |
| Trouvé | 52,99 | 7,96 | 24,75 | 14,34 |

Le spectre ¹H RMN est conforme à la structure.

### EXEMPLE 5

### Diamino-2,4 n-octyloxy-6 pyrimidine oxyde-3

La préparation est effectuée suivant le mode opératoire décrit à l'exemple 3 avec le n-octanol.
Température : 110°C Temps : 17 heures
Recristallisation : méthanol
Rendement : 13,7%
F = 138-139°C.

| Analyse élémentaire : C₁₂H₂₂N₄O₂; M = 254. | | | | |
|---|---|---|---|---|
| | C | H | N | O |
| Calculé | 56,69 | 8,66 | 22,05 | 12,60 |
| Trouvé | 56,44 | 8,63 | 21,87 | 12,64 |

Les spectres de RMN ¹H et ¹³C et de masse sont conformes à la structure.

### EXEMPLE 6

### Diamino-2,4 n-dodécyloxy-6 pyrimidine oxyde-3

La préparation est effectuée suivant le mode opératoire décrit à l'exemple 3 avec le dodécanol.
Température : 110°C Temps : 20 heures
Rendement : 25%
F = 136°C.

| Analyse élémentaire : C₁₆H₃₀N₄O₂; M = 310. | | | | |
|---|---|---|---|---|
| | C | H | N | O |
| Calculé | 61,93 | 9,68 | 18,06 | 10,32 |
| Trouvé | 62,01 | 9,76 | 17,96 | 10,45 |

Les spectres de RMN ¹H et ¹³C et de masse sont conformes à la structure.

### EXEMPLE 7

### Diamino-2,4 (éthyl-2 hexyloxy)-6 pyrimidine oxyde-3

La préparation est effectuée suivant le mode opératoire décrit à l'exemple 3 avec l'éthyl-2 hexanol.
Température : 130°C Temps : 24 heures
Recristallisation : mélange dichlorométhane/méthanol
Rendement < 10%
F = 178°-180°C.

| Analyse élémentaire : C₁₂H₂₂N₄O₂; M = 254. | | | | |
|---|---|---|---|---|
| | C | H | N | O |
| Calculé | 56,69 | 8,66 | 22,05 | 12,60 |
| Trouvé | 56,62 | 8,75 | 22,08 | 12,63 |

Les spectres de RMN ¹³C et de masse sont conformes à la structure.

### EXEMPLE 8

### Diamino-2,4 (n-héxenyl-5 oxy)-6 pyrimidine oxyde-3

La préparation est effectuée suivant le mode opératoire décrit à l'exemple 3 avec l'alcool n-hexen-5 ol-1.
Température : 115°C Temps : 20 heures
Recristallisation : méthanol
Rendement : 37%
F = 98-102°C.

| Analyse élémentaire : C₁₀H₁₆N₄O₂; M = 224. | | | | |
|---|---|---|---|---|
| | C | H | N | O |
| Calculé | 53,57 | 7,14 | 25,00 | 14,28 |
| Trouvé | 53,68 | 7,15 | 25,04 | 14,40 |

Les spectres de RMN ¹³C et de masse sont conformes à la structure.

### EXEMPLE 9

### Diamino-2,4 (undécényl-10 oxy)-6 pyrimidine oxyde-3

La préparation est effectuée suivant le mode opératoire décrit à l'exemple 3 avec l'alcool undécén-10 ol-1.
Température : 110°C Temps = 27 heures
Recristallisation : acétonitrile
Rendement : 23%
F = 131°C.

| Analyse élémentaire : C₁₅H₂₆N₄O₂; M = 294. | | | | |
|---|---|---|---|---|
| | C | H | N | O |
| Calculé | 61,22 | 8,84 | 19,05 | 10,88 |
| Trouvé | 61,23 | 8,86 | 18,85 | 11,14 |

Les spectres de RMN ¹H, ¹³C et de masse sont conformes à la structure.

### EXEMPLE 10

### Diamino-2,4 (phényl-2 éthyloxy)-6 pyrimidine oxyde-3

La préparation est effectuée suivant le mode opératoire décrit à l'exemple 1 avec l'alcool phényl-2 éthylique.
Température : 115°-120°C Temps : 11 heures
Recristallisation : méthanol
Rendement : 18%
F = 204°C

| Analyse élémentaire : C₁₂H₁₄N₄O₂; M = 246. | | | | |
|---|---|---|---|---|
| | C | H | N | O |
| Calculé | 58,54 | 5,69 | 22,76 | 13,01 |
| Trouvé | 58,51 | 5,69 | 22,58 | 13,16 |

Les spectres de RMN ¹³C et de masse sont conformes à la structure.

### EXEMPLE 11

### Amino-2 acétamido-4 butyloxy-6 pyrimidine oxyde-3

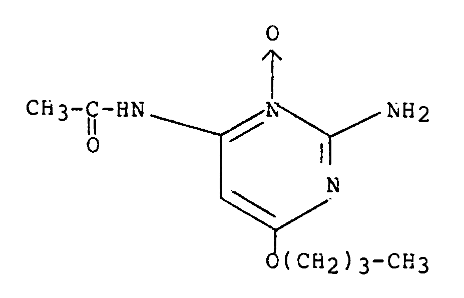

Dans un ballon tricol de 500 ml muni d'un thermomètre, d'une agitation, d'un réfrigérant et d'une arrivée d'argon, on introduit 4,76 g de diamino-2,4 n-butyloxy-6 pyrimidine oxyde-3 et 300 ml de dichlorométhane. On porte au reflux du solvant et on ajoute lentement une solution de 2,44 q d'anhydride acétique dans 30 ml de dichlorométhane.

Après 10 minutes d'introduction, on refroidit à température ambiante et on transvase en ampoule à décanter.

On lave la phase organique jusqu'à neutralité puis on concentre la solution sous vide.

On ajoute de l'hexane jusqu'à l'apparition d'un précipité que l'on filtre. Après dissolution dans le dichlorométhane, on le précipite une seconde fois à l'hexane. Le précipité est recristallisé dans un mélange acétone/dichlorométhane puis séché sous vide.

On obtient 2 g d'un composé fondant à 184°C et conforme à la structure attendue (spectres de masse et ¹H RMN).

| Analyse élémentaire : C₁₀H₁₆N₄O₃; M = 240. | | | | |
|---|---|---|---|---|
| | C | H | N | O |
| Calculé | 50,00 | 6,66 | 23,33 | 20,00 |
| Trouvé | 50,04 | 6,72 | 23,34 | 20,11 |

### EXEMPLE 12

### Diacétamido-2,4 n-butyloxy-6 pyrimidine oxyde-3

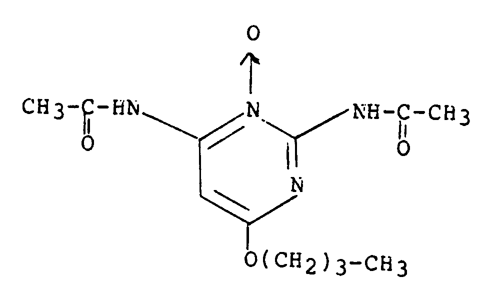

Dans un ballon tricol de 50 ml muni d'un thermomètre, d'une agitation et d'une arrivée d'argon, on introduit 2 g de diamino-2,4 n-butyloxy-6 pyrimidine oxyde-3, 20 ml de dichlorométhane et 5,7 ml de triéthylamine.

On refroidit à -10°C et on ajoute en 30 minutes 3,2 g de chlorure d'acétyle sans dépasser 0°C.

On rajoute 10 ml de dichlorométhane et on maintient encore 2 heures 30 minutes à cette température. On transvase en ampoule à décanter et on lave la phase organique jusqu'à neutralité. On concentre sous vide puis on ajoute de l'hexane jusqu'à obtention d'un précipité que l'on filtre. Après recristallisation dans un mélange acétone/dichlorométhane, on obtient 1,5 g d'un composé pur fondant à 168°C dont les spectres de masse et ¹H RMN confirment la structure attendue.

| Analyse élémentaire : C₁₂H₁₈N₄O₄; M = 282. | | | | |
|---|---|---|---|---|
| | C | H | N | O |
| Calculé | 51,06 | 6,38 | 19,86 | 22,69 |
| Trouvé | 51,19 | 6,41 | 19,93 | 22,87 |

### EXEMPLE 15

### N-(amino-2 oxyde-3 butyloxy-6 pyrimidinyl-4),N′-diméthylurée

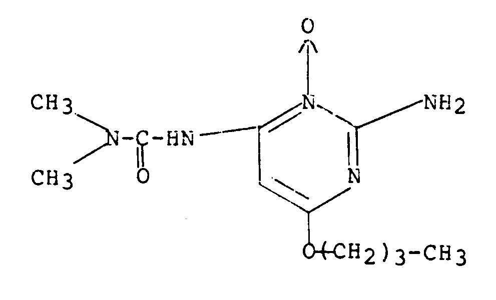

Dans un ballon tricol de 50 ml muni d'un réfrigérant, d'un thermomètre et d'une arrivée d'argon, on introduit 2 g de diamino-2,4 n-butyloxy-6 pyrimidine oxyde-3, puis 10 ml de diméthylsulfoxyde.

On amène à 50°C sous agitation puis on introduit en 5 minutes 1,19 g de chlorure de N-diméthylcarbamoyle.

On agite encore 2 heures 30 minutes à 50°C, puis on verse le mélange réactionnel dans 100 ml d'eau. On extrait ensuite par quatre fois 50 ml de dichlorométhane. Les fractions organiques réunies sont évaporées à sec et le résidu repris à l'éther éthylique.

On élimine un insoluble et les eaux-mères sont passées sur une colonne de silice.

Après élution par un mélange acétate d'éthyle (90)/méthanol (10), on récupère 1 g de composé fondant à 122°C dont les spectres de masse et ¹H RMN montrent qu'il répond à la structure attendue.

| Analyse élémentaire : C₁₁H₁₉N₅O₃; M = 269. | | | | |
|---|---|---|---|---|
| | C | H | N | O |
| Calculé | 49,07 | 7,06 | 26,02 | 17,84 |
| Trouvé | 48,69 | 6,80 | 25,75 | 18,56 |

### EXEMPLE DE COMPOSITION 1

On prépare la composition suivante :

| | |
|---|---|
| - Diamino-2,4 (n-butyloxy)-6 pyrimidine oxyde-3 | 3,0 g |
| - Hydroxypropylcellulose vendue sous la dénomination commerciale "KLUCEL G" par la Société HERCULES | 3,0 g |
| - Butyl hydroxy toluène | 0,1 g |
| - Mélange éthanol/eau (50:50) | qsp 100,0 g |

Cette composition se présente sous forme d'un gel.

1 à 2 g de cette composition sont appliqués sur les zones alopéciques du cuir chevelu, éventuellement accompagné d'un massage pour favoriser sa pénétration, à raison de une à deux applications par jour, pendant trois mois de traitement.

### EXEMPLE DE COMPOSITION 2

On prépare la composition suivante :

| | |
|---|---|
| - Diamino-2,4 (n-butyloxy)-6 pyrimidine oxyde-3 | 6,0 g |
| - Mélange éthanol absolu/propylène glycol (95:5) | qsp 100,0 g |

### EXEMPLE DE COMPOSITION 3

On prépare la composition suivante :

| | |
|---|---|
| - Diamino-2,4 (n-butyloxy)-6 pyrimidine oxyde-3 | 4,0 g |
| - Propylène glycol | 20,0 g |
| - Ethanol | 50,0 g |
| - Eau | qsp 100,0 g |

### EXEMPLE DE COMPOSITION 4

On prépare la composition suivante :

| | |
|---|---|
| - Diamino-2,4 (n-butyloxy)-6 pyrimidine oxyde-3 | 2,0 g |
| - Méthyl hydroxy propyl cellulose vendue sous la dénomination commerciale "METHOCEL F" par la Société DOW CHEMICAL | 1,0 g |
| - Ethanol | 36,0 g |
| - Eau | qsp 100,0 g |

### EXEMPLE DE COMPOSITION 5

On prépare la composition suivante :

| | |
|---|---|
| - Diamino-2,4 éthyloxy-6 pyrimidine oxyde-3 | 2,5 g |
| - Propylène glycol | 20,0 g |
| - Ethanol | 40,0 g |
| - Eau | qsp 100,0 g |

Cette composition se présente sous forme de lotion active contre la chute des cheveux.

### EXEMPLE DE COMPOSITION 6

On prépare un shampooing destiné au traitement de la chute des cheveux, de composition suivante :

| | |
|---|---|
| - Diamino-2,4 n-butyloxy-6 pyrimidine oxyde-3 | 2,0 g |
| - Tensio-actif non-ionique obtenu par | 13,0 g MA |
| condensation de 3,5 moles de glycidol sur un α-diol en C₁-C₁₄ selon le brevet français n° 71-17206 | |
| - Complexant (MASCOLATE de Fe) | 0,2 g |
| - Conservateurs | 0,5 g |
| - Eau | qsp 100,0 g |

### EXEMPLE DE COMPOSITION 7

On prépare la composition suivante :

| | |
|---|---|
| - Diamino-2,4 n-butyloxy-6 pyrimidine oxyde-3 | 0,3 g |
| - Hexadécyléther de diglycérol de formule C₁₆H₃₃-O(CH₂-CHOH-CH₂-O)₂H | 3,8 g |
| - Cholestérol | 3,8 g |
| - Acylglutamate HS11 vendu par la Société AJINOMOTO | 0,4 g |
| - Conservateurs | 0,4 g |
| - Eau | qsp 100,0 g |

Cette composition se présente sous forme d'émulsion vésiculaire.

### EXEMPLE DE COMPOSITION 8

On prépare une lotion de composition suivante :

| | |
|---|---|
| - Diamino-2,4(phényl-2 éthyloxy)-6 pyrimidine oxyde-3 | 5,0 g |
| - Propylèneglycol | 22,8 g |
| - Ethanol | 55,1 g |
| - Eau | qsp 100,0 g |

### EXEMPLE DE COMPOSITION 9

On prépare une lotion de composition suivante :

| | |
|---|---|
| - Diamino-2,4(phényl-2 éthyloxy)-6 pyrimidine oxyde-3 | 4,0 g |
| - Propylèneglycol | 6,45 g |
| - Ethanol absolu | qsp 100,0 g |

### EXEMPLE DE COMPOSITION 10

On prépare une lotion de composition suivante :

| | |
|---|---|
| - Diamino-2,4(phényl-2 éthyloxy)-6 pyrimidine oxyde-3 | 2,5 g |
| - Ethanol | 50,0 g |
| - Eau | qsp 100,0 g |

1 à 2 ml de ces lotions sont appliqués sur les zones alopéciques du cuir chevelu; ces applications, éventuellement accompagnées par un massage pour favoriser la pénétration, étant effectuées une ou deux fois par jour.

## Revendications (Revendications pour l'(les) Etat(s) contractant(s) suivant(s): AT, BE, CH, DE, FR, GB, IT, LI, NL, SE)

1. Composé caractérisé par le fait qu'il répond à la formule : 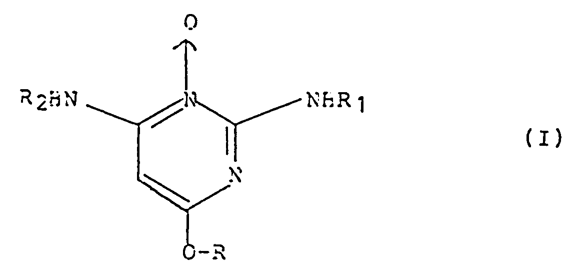 dans laquelle :
R₁ et R₂ désignent, indépendamment l'un de l'autre, un atome d'hydrogène ou bien un groupement carbamoyle de formule : 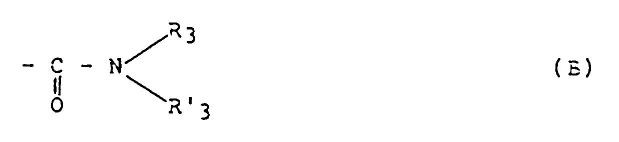 avec R'₃ = H ou R₃:
ou un groupement acyle de formule : 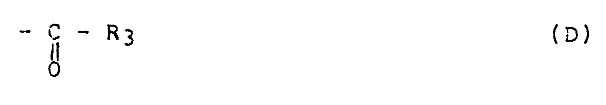 dans laquelle R₃ désigne un radical alkyle linéaire ou ramifié en C₁-C₁₈, un groupement alcényle en C₂-C₁₈, un groupement cycloalkyle en C₅-C₈; R₃ peut également désigner un radical aryle ou aralkyle répondant à la formule : 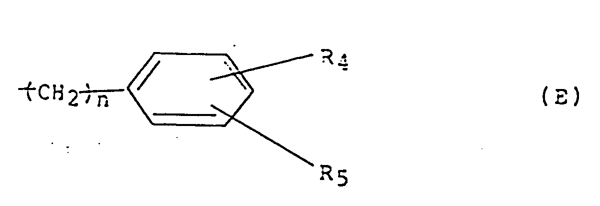 dans laquelle :
n est compris entre 0 et 4;
R₄ et/ou R₅, indépendamment l'un de l'autre, désignent hydrogène, un groupement alkyle inférieur en C₁-C₆, un groupement hydroxyle, alcoxy en C₁-C₆, un atome d'halogène ou un groupement CF₃;
R désigne un radical alkyle, linéaire ou ramifié en C₁-C₁₈, alcényle en C₂-C₁₈, un radical cycloalkyle en C₄-C₆ pouvant porter une insaturation ou un radical alkyle en C₁-C₆ portant un groupement phényle ou pyridine.

2. Composé selon la revendication 1, caractérisé par le fait que le radical R est choisi parmi les groupements méthyle, éthyle, butyle, isobutyle, n-hexyle, n-octyle, n-décyle, lauryle, éthyl-2 hexyl, n-héxényl-5, undécényl-10, cyclohexyle, phénéthyle ou benzyle.

3. Composé selon la revendication 1 ou 2, caractérise par le fait qu'il s'agit du diamino-2,4 (n-butyloxy)-6 pyrimidine oxyde-3 et ses sels cosmétiquement ou pharmaceutiquement acceptables.

4. Composé selon la revendication 1 ou 2, caractérisé par le fait qu'il s'agit du diamino-2,4 éthyloxy-6 pyrimidine oxyde-3 ou ses sels cosmétiquement ou pharmaceutiquement acceptables.

5. Composé de la famille des diamino-2,4 pyrimidine oxyde-3 selon la revendication 1 choisi parmi la diamino-2,4 méthoxy-6 pyrimidine oxyde-3, la diamino-2,4 n-hexyloxy-6 pyrimidine oxyde-3, la diamino-2,4 n-octyloxy-6 pyrimidine oxyde-3, la diamino-2,4 n-dodécyloxy-6 pyrimidine oxyde-3, la diamino-2,4(éthyl-2 hexyloxy)-6 pyrimidine oxyde-3, la diamino-2,4(n-hexenyl-5 oxy)-6 pyrimidine oxyde-3, la diamino-2,4(undécényl-10 oxy)-6 pyrimidine oxyde-3, la diamino-2,4(phényl-2 éthyloxy)-6 pyrimidine oxyde-3 ou les sels cosmétiquement ou pharmaceutiquement acceptables.

6. Composé selon la revendication 1, caractérisé par le fait qu'il est choisi parmi l'amino-2 acétamido-4 butyloxy-6 pyrimidine oxyde-3, le diacétamido-2,4 butyloxy-6 pyrimidine oxyde-3, la N(amino-2 oxyde-3 butyloxy-6 pyrimidinyl-4)N'-diméthylurée et ses sels cosmétiquement ou pharmaceutiquement acceptables.

7. Composition destinée à être utilisée en application topique, caractérisée par le fait qu'elle contient, dans un milieu physiologiquement acceptable, au moins un composé tel que défini dans l'une quelconque des revendications 1 à 6.

8. Composition selon la revendication 7, caractérisée par le fait qu'elle se présente sous forme d'onguent, de teinture, de crème, de pommade, de poudre, de timbre, de tampon imbibé, de solution, d'émulsion, d'émulsion vésiculaire, de lotion, de gel, de spray ou de suspension anhydre ou aqueuse, en vue de son application pharmaceutique et qu'elle contient au moins un composé tel que défini dans l'une quelconque des revendications 1 à 6.

9. Composition selon la revendication 7 ou 8, caractérisée par le fait que les composés de formule (I) sont présents à des concentrations comprises entre 0,1 et 10% en poids par rapport au poids total de la composition et en particulier entre 0,2 et 5% en poids.

10. Composition destinée à être utilisée en cosmétique, telle que définie dans la revendication 7, caractérisée par le fait qu'elle se présente sous forme de lotion, de gel, de savon, de shampooing, d'aérosol ou de mousse et qu'elle contient, dans un support acceptable sur le plan cosmétique, au moins un composé tel que défini dans l'une quelconque des revendications 1 à 6, à une concentration comprise entre 0,01 et 7,5% en poids.

11. Composition selon l'une quelconque des revendications 7 à 10, caractérisée par le fait qu'elle contient, en plus des agents hydratants, des agents antiséborrhéiques.

12. Composition selon l'une quelconque des revendications 7 à 11, caractérisée par le fait qu'elle contient écalement des agents améliorant encore l'activité des composés de formule (I) au niveau de la repousse et/ou du freinage de la chute des cheveux.

13. Composition selon la revendication 12, caractérisée par le fait qu'elle contient à titre d'agents améliorant encore l'activité de la repousse et/ou du freinage de la chute des cheveux, des esters d'acide nicotinique, des agents anti-inflammatoires stéroïdiens ou non stéroïdiens, des rétinoïdes, des agents antibactériens, des agents antagonistes du calcium, des hormones, des agents antiandrogènes, des capteurs de radicaux OH.

14. Composition selon la revendication 12, caractérisée par le fait qu'elle contient à titre de composés améliorant encore l'activité sur la repousse et/ou le freinage de la chute des cheveux, des composés choisis parmi le diazoxyde, la spiroxazone, des phospholipides, des acides linolénique et linoléique, l'acide salicylique et ses dérivés, des acides hydroxycarboxyliques ou cétocarboxyliques, leurs esters, des lactones et leurs sels correspondants, l'anthraline ou le trihydroxy-1,8,9 anthracène, les caroténoïdes, les acides eicosatétraynoïques-5,8,11,14 eicosatriynoïques-5,8,11, leurs esters et amides.

15. Composition selon l'une quelconque des revendications 7 à 14, caractérisée par le fait que le milieu physiologiquement acceptable est constitué par de l'eau, un mélange d'eau et d'un ou plusieurs solvant(s) organique(s) ou par un mélange de solvants organiques, les solvants organiques étant pharmaceutiquement ou cosmétiquement acceptables.

16. Composition selon la revendication 15, caractérisée par la fait que les solvants sont choisis parmi les alcools inférieurs en C₁-C₄, les alkylène glycols, les alkyléthers de mono- et ce dialkylène glycol.

17. Composition selon l'une quelconque des revendications 7 à 16, caractérisée par la fait que le milieu physiologiquement acceptable est épaissi au moyen d'agents épaississants et/ou gélifiants et contient des agents conservateurs, des agents stabilisants, des agents régulateurs de pH, des agents modificateurs de pression osmotique, des agents émulsifiants, des filtres UVA et UVB, des agents anti-oxydants.

18. Composition selon l'une quelconque des revendications 7 à 17, caractérisée par le fait qu'elle contient également des agents tensio-actifs choisis parmi les agents tensio-actifs non ioniques et amphotères.

19. Composition selon l'une quelconque des revendications 7 à 18, pour son application comme médicament destiné a être utilisé dans le traitement thérapeutique de la chute des chevaux.

20. Procédé de traitement cosmétique des cheveux ou du cuir chevelu, caractérisé par le fait que l'on applique la composition telle que définie dans l'une quelconque des revendications 7 à 18.

21. Utilisation de la composition telle que définie dans l'une quelconque des revendications 7 à 18, pour la préparation d'un médicament destiné à être utilisé dans le traitement thérapeutique de la chute des cheveux.

22. composé diamino-2,4 (trifluoro-2 éthyloxy)-6 pyrimidine oxyde-3

## Revendications (Revendications pour l'(les) Etat(s) contractant(s) suivant(s): ES)

1. Procédé de préparation d'un composé répondant à la formule : 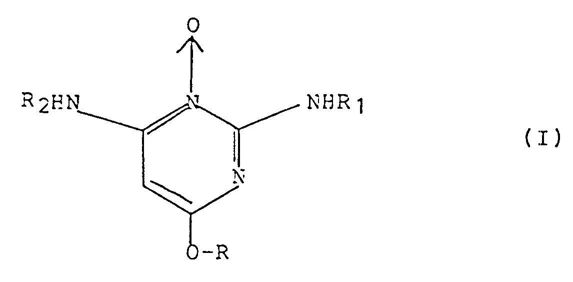 dans laquelle :
R₁ et R₂ désignent, indépendamment l'un de l'autre, un atome d'hydrogène ou bien un groupement carbamoyle de formule : 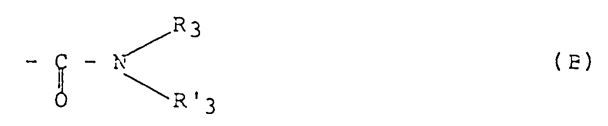 avec R'₃ = H ou R₃
ou un groupement acyle de formule : 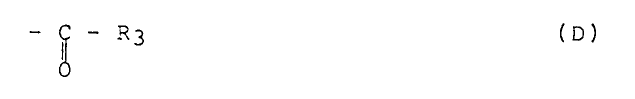 dans laquelle R₃ désigne un radical alkyle linéaire ou ramifié en C₁-C₁₈, un groupement alcényle en C₂-C₁₈, un groupement cycloalkyle en C₅-C₈; R₃ peut également désigner un radical aryle ou aralkyle répondant à la formule : 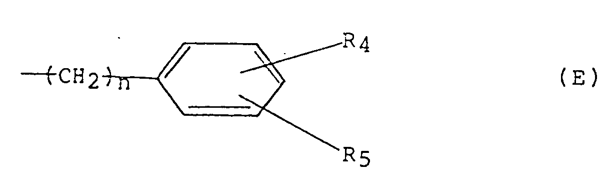 dans laquelle :
n est compris entre 0 et 4;
R₄ et/ou R₅, indépendamment l'un de l'autre, désignent hydrogène, un groupement alkyle inférieur en C₁-C₆, un groupement hydroxyle, alcoxy en C₁-C₆, un atome d'halogène ou an groupement CF₃;
R désigne un radical alkyle, linéaire ou ramifié en C₁-C₁₈, alcényle en C₂-C₁₈, un radical cycloalkyle en C₄-C₆ pouvant porter une insaturation ou un radical alkyle en C₁-C₆ portant un groupement phényle ou pyridine caractérisé par le fait que :
(i) lorsque R₁ et R₂ désignent hydrogène, on part du diamino-2,4(dichloro-2,4 phénoxy)-6 pyrimidine oxyde-3 ou du diamino-2,4 chloro-6 pyrimidine oxyde-3 que l'on fait réagir avec un alcoolate de formule RO^{⊖}Y^{⊕} où R a la spécification indiquée ci-dessus et Y est un cation alcalin tel que le sodium, le potassium, le lithium;
(ii) lorsque R₁ et R₂ sont des groupements carbamoyle, ou acyle, ils sont obtenus à partir ces dérivés diamino-2,4 alcoxy-6 pyrimidine oxyde-3 correspondants :
- l'obtention de dérivés pour lesquels R₁ et R₂ sont des groupements carbamoyle, est réalisée en faisant réagir un chlorure de carbamoyle sur le dérivé diamino-2,4 alcoxy-6 pyrimidine oxyde-3 correspondant dans un solvant polaire, à une température comprise entre 0 et 100°C;
- l'obtention de dérivés pour lesquels R₁ et R₂ sont des groupements acyle, est réalisée en faisant réagir un chlorure d'acide ou un anhydride sur le dérivé diamino-2,4 alcoxy-6 pyrimidine 3-oxyde correspondant dans un solvant polaire aprotique, en présence d'une amine tertiaire, a une température comprise entre 0 et 50°C.

2. Procédé selon la revendication 1, caractérisé par le fait que le radical R est choisi parmi les groupements méthyle, éthyle, butyle, isobutyle, n-hexyle, n-octyle, n-décyle, lauryle, éthyl-2 hexyle, n-héxényl-5, undécényl-10, cyclohexyle, phénéthyle ou benzyle.

3. Procédé selon la revendication 1 ou 2, caractérisé par le fait qu'il s'agit du diamino-2,4 (n-butyloxy)-6 pyrimidine oxyde-3 et ses sels cosmétiquement ou pharmaceutiquement acceptables.

4. Procédé selon la revendication 1 ou 2, caractérisé par le fait qu'il s'agit du diamino-2,4 éthyloxy-6 pyrimidine oxyde-3 ou ses sels cosmétiquement ou pharmaceutiquement acceptables.

5. Procédé selon la revendication 1, caractérisé par le fait que le composé appartient à la famille des diamino-2,4 pyrimidine oxyde-3 choisi parmi la diamino-2,4 méthoxy-6 pyrimidine oxyde-3, la diamino-2,4 n-hexyloxy-6 pyrimidine oxyde-3, la diamino-2,4 n-octyloxy-6 pyrimidine oxyde-3, la diamino-2,4 n-dodécyloxy-6 pyrimidine oxyde-3, la diamino-2,4(éthyl-2 hexyloxy)-6 pyrimidine oxyde-3, la diamino-2,4(n-hexenyl-5 oxy)- 6 pyrimidine oxyde-3, la diamino-2,4(undécényl-10 oxy)-6 pyrimidine oxyde-3, la diamino-2,4(phényl-2 éthyloxy)-6 pyrimidine oxyde-3 ou les sels cosmétiquement ou pharmaceutiquement acceptables.

6. Procédé selon la revendication 1, caractérisé par le fait que le composé préparé est choisi parmi l'amino-2 acétamido-4 butyloxy-6 pyrimidine oxyde-3, le diacétamido-2,4 butyloxy-6 pyrimidine oxyde-3, la N(amino-2 oxyde-3 butyloxy-6 pyrimidinyl-4)N'-diméthylurée et ses sels cosmétiquement ou pharmaceutiquement acceptables.

7. Composition destinée à être utilisée en application topique, caractérisée par le fait qu'elle contient, dans un milieu physiologiquement acceptable, au moins un composé tel que défini dans l'une quelconque des revendications 1 à 6.

8. Composition selon la revendication 7, caractérisée par le fait qu'elle se présente sous forme d'onguent, de teinture, de crème, de pommade, de poudre, de timbre, de tampon imbibé, de solution, d'émulsion, d'émulsion vésiculaire, de lotion, de gel, de spray ou de suspension anhydre ou aqueuse, en vue de son application pharmaceutique et qu'elle contient au moins un composé tel que défini dans l'une quelconque des revendications 1 à 6.

9. Composition selon la revendication 7 ou 8, caractérisée par le fait que les composés de formule (I) sont présents à des concentrations comprises entre 0,1 et 10% en poids par rapport au poids total de la composition et en particulier entre 0,2 et 5% en poids.

10. Composition destinée à être utilisée en cosmétique, telle que définie dans la revendication 7, caractérisée par le fait qu'elle se présente sous forme de lotion, de gel, de savon, de shampooing, d'aérosol ou de mousse et qu'elle contient, dans un support acceptable sur le plan cosmétique, au moins un composé tel que défini dans l'une quelconque des revendications 1 à 6, à une concentration comprise entre 0,01 et 7,5% en poids.

11. Composition selon l'une quelconque des revendications 7 à 10, caractérisée par le fait qu'elle contient, en plus des agents hydratants, des agents antiséborrhéiques.

12. Composition selon l'une quelconque des revendications 7 à 11, caractérisée par le fait qu'elle contient également des agents améliorant encore l'activité des composés de formule (I) au niveau de la repousse et/ou du freinage de la chute des cheveux.

13. Composition selon la revendication 12, caractérisée par le fait qu'elle contient à titre d'agents améliorant encore l'activité de la repousse et/ou du freinage de la chute des cheveux, des esters d'acide nicotinique, des agents anti-inflammatoires stéroïdiens ou non stéroïdiens, des rétinoïdes, des agents antibactériens, des agents antagonistes du calcium, des hormones, des agents antiandrogènes, des capteurs de radicaux OH.

14. Composition selon la revendication 12, caractérisée par le fait qu'elle contient à titre de composés améliorant encore l'activité sur la repousse et/ou le freinage de la chute des cheveux, des composés choisis parmi le diazoxyde, la spiroxazone, des phospholipides, des acides linolénique et linoléique, l'acide salicylique et ses dérivés, des acides hydroxycarboxyliques ou cétocarboxyliques, leurs esters, des lactones et leurs sels correspondants, l'anthraline ou le trihydroxy-1,8,9 anthracène, les caroténoïdes, les acides eicosatétraynoïques-5,8,11,14 eicosatriynoïques-5,8,11, leurs esters et amides.

15. Composition selon l'une quelconque des revendications 7 à 14, caractérisée par le fait que le milieu physiologiquement acceptable est constitué par de l'eau, un mélange d'eau et d'un ou plusieurs solvant(s) organique(s) ou par un mélange de solvants organiques, les solvants organiques étant pharmaceutiquement ou cosmétiquement acceptables.

16. Composition selon la revendication 15, caractérisée par le fait que les solvants sont choisis parmi les alcools inférieurs en C₁-C₄, les alkylène glycols, les alkyléthers de mono- et de dialkylène glycol.

17. Composition selon l'une quelconque des revendications 7 à 16, caractérisée par le fait que le milieu physiologiquement acceptable est épaissi au moyen d'agents épaississants et/ou gélifiants et contient des agents conservateurs, des agents stabilisants, des agents régulateurs de pH, des agents modificateurs de pression osmotique, des agents émulsifiants, des filtres UVA et UVB, des agents anti-oxydants.

18. Composition selon l'une quelconque des revendications 7 à 17, caractérisée par le fait qu'elle contient également des agents tensio-actifs choisis parmi les agents tensio-actifs non ioniques et amphotères.

19. Composition selon l'une quelconque des revendications 7 à 18, pour son application comme médicament destiné à être utilisé dans le traitement thérapeutique de la chute des cheveux.

20. Procédé de préparation d'une composition destinée à être utilisée en application topique, caractérisé par le fait que l'on introduit dans un milieu physiologiquement acceptable dans des proportions de 0,1 à 10% en poids par rapport au poids total de la composition, au moins un composé de formule (I) : 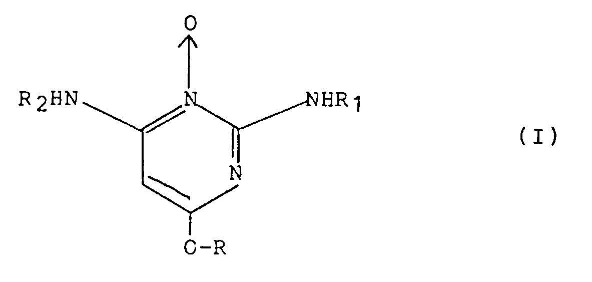 dans laquelle :
R₁ et R₂ désignent, indépendamment l'un de l'autre, un atome d'hydrogène ou bien un groupement carbamoyle de formule : 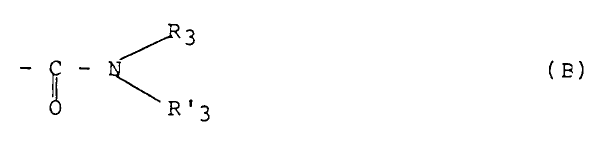 avec R'₃ = H ou R₃
ou un groupement acyle de formule : 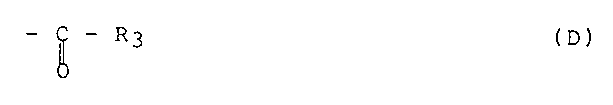 dans laquelle R₃ désigne un radical alkyle linéaire ou ramifié en C₁-C₁₈, un groupement alcényle en C₂-C₁₈, un groupement cycloalkyle en C₅-C₈; R₃ peut également désigner un radical aryle ou aralkyle répondant à la formule : 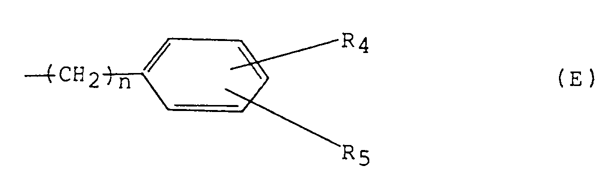 dans laquelle :
n est compris entre 0 et 4;
R₄ et/ou R₅, indépendamment l'un de l'autre, désignent hydrogène, un groupement alkyle inférieur en C₁-C₆, un groupement hydroxyle, alcoxy en C₁-C₆, un atome d'halogène ou un groupement CF₃;
R désigne un radical alkyle, linéaire ou ramifié en C₁-C₁₈, alcényle en C₂-C₁₈, un radical cycloalkyle en C₄-C₆ pouvant porter une insaturation ou un radical alkyle en C₁-C₆ portant un groupement phényle ou pyridine.

21. composé diamino-2,4 (trifluoro-2 éthyloxy)-6 pyrimidine oxyde-3

## Claims (Claims for the following Contracting State(s): AT, BE, CH, DE, FR, GB, IT, LI, NL, SE)

1. Compound characterized in that it corresponds to the formula: 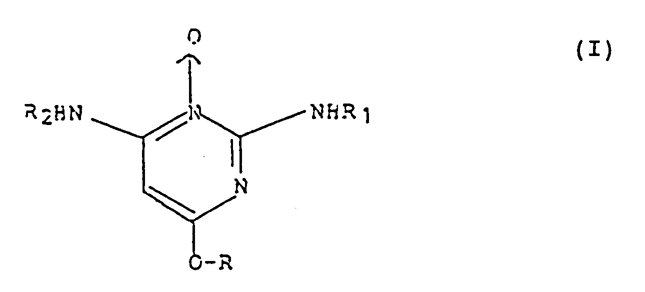 in which:
R₁ and R₂ denote, independently of each other, a hydrogen atom or a carbamoyl group of formula: 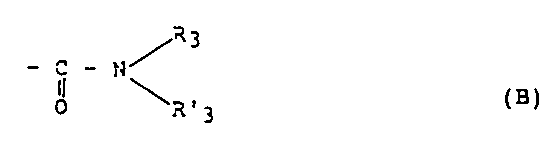 with R'₃ = H or R₃
or an acyl group of formula: 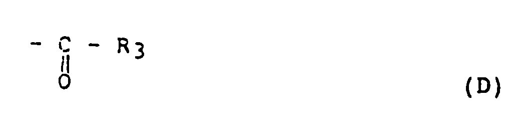 in which R₃ denotes a linear or branched C₁-C₁₈ alkyl radical, a C₂-C₁₈ alkenyl group or a C₅-C₈ cycloalkyl group; R₃ may also denote an aryl or aralkyl radical corresponding to the formula: 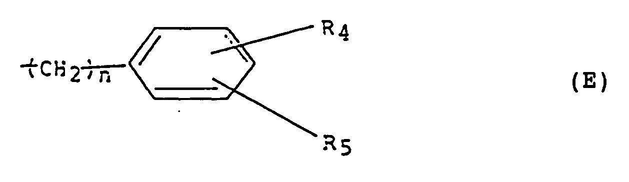 in which:
n is between 0 and 4;
R₄ and/or R₅, independently of each other, denote hydrogen, a lower C₁-C₆ alkyl group, a hydroxyl or C₁-C₆ alkoxy group, a halogen atom or a CF₃ group;
R denotes a linear or branched C₁-C₁₈ alkyl or C₂-C₁₈ alkenyl radical, a C₄-C₆ cycloalkyl radical capable of bearing an unsaturation or a C₁-C₆ alkyl radical bearing a phenyl or pyridine group.

2. Compound according to Claim 1, characterized in that the radical R is chosen from methyl, ethyl, butyl, isobutyl, n-hexyl, n-octyl, n-decyl, lauryl, 2-ethylhexyl, 5-n-hexenyl, 10-undecenyl, cyclohexyl, phenethyl or benzyl groups.

3. Compound according to Claim 1 or 2, characterized in that it is 2,4-diamino-6-(n-butyloxy)pyrimidine 3-oxide and its cosmetically or pharmaceutically acceptable salts.

4. Compound according to Claim 1 or 2, characterized in that it is 2,4-diamino-6-ethyloxypyrimidine 3-oxide or its cosmetically or pharmaceutically acceptable salts.

5. Compound of the group of 2,4-diaminopyrimidine 3-oxides according to Claim 1, chosen from 2,4-diamino-6-methoxypyrimidine 3-oxide, 2,4-diamino-6-n-hexyloxypyrimidine 3-oxide, 2,4-diamino-6-n-octyloxypyrimidine 3-oxide, 2,4-diamino-6-n-dodecyloxypyrimidine 3-oxide, 2,4-diamino-6-(2-ethylhexyloxy)pyrimidine 3-oxide, 2,4-diamino-6-(5-n-hexenyloxy)pyrimidine-3-oxide, 2,4-diamino-6-(10-undecenyloxy)pyrimidine 3-oxide, 2,4-diamino-6-(2-phenylethyloxy)pyrimidine 3-oxide, or cosmetically or pharmaceutically acceptable salts.

6. Compound according to Claim 1, characterized in that it is chosen from 2-amino-4-acetamido-6-butyloxypyrimidine 3-oxide, 2,4-diacetamido-6-butyloxypyrimidine 3-oxide and N-(2-amino-6-butyloxy-4-pyrimidinyl)-N'-dimethylurea 3-oxide and its cosmetically or pharmaceutically acceptable salts.

7. Composition intended to be employed in topical application, characterized in that it contains, in a physiologically acceptable medium, at least one compound such as defined in any one of Claims 1 to 6.

8. Composition according to Claim 7, characterized in that it is in the form of an ointment, tincture, cream, pomade, powder, patch, impregnated pad, solution, emulsion, vesicular emulsion, lotion, gel, spray or anhydrous or aqueous suspension, with a view to its pharmaceutical application, and in that it contains at least one compound such as defined in any one of Claims 1 to 6.

9. Composition according to Claim 7 or 8, characterized in that the compounds of formula (I) are present in concentrations of between 0.1 and 10 % by weight relative to the total weight of the composition, and in particular between 0.2 and 5 % by weight.

10. Composition intended to be employed in cosmetics, such as defined in Claim 7, characterized in that it is in the form of a lotion, gel, soap, shampoo, aerosol or foam and that it contains, in a cosmetically acceptable carrier, at least one compound such as defined in any one of Claims 1 to 6, in a concentration of between 0.01 and 7.5 % by weight.

11. Composition according to any one of Claims 7 to 10, characterized in that, in addition to hydrating agents, it contains antiseborrhoeic agents.

12. Composition according to any one of Claims 7 to 11, characterized in that it also contains agents which further improve the activity of the compounds of formula (I) relating to the fresh growth and/or delaying the loss of hair.

13. Composition according to Claim 12, characterized in that it contains, by way of agents which further improve the activity of the fresh growth and/or delaying the loss of hair, nicotinic acid esters, steroid or nonsteroid antiinflammatory agents, retinoids, antibacterial agents, calcium antagonist agents, hormones, antiandrogen agents or scavengers for OH radicals.

14. Composition according to Claim 12, characterized in that it contains, by way of compounds further improving the activity relating to the fresh growth and/or delaying the loss of hair, compounds chosen from diazoxide, spiroxazone, phospholipids, linolenic and linoleic acids, salicylic acid and its derivatives, hydrocarboxylic or ketocarboxylic acids, their esters, lactones and their corresponding salts, anthralin or 1,8,9-trihydroxyanthracene, carotenoids, 5,8,11,14-eicosatetraynoic and 5,8,11-eicosatriynoic acids and their esters and amides.

15. Composition according to any one of Claims 7 to 14, characterized in that the physiologically acceptable medium consists of water, a mixture of water and of one or more organic solvent(s) or of a mixture of organic solvents, the organic solvents being pharmaceutically or cosmetically acceptable.

16. Composition according to Claim 15, characterized in that the solvents are chosen from lower C₁-C₄ alcohols, alkylene glycols and mono- and dialkylene glycol alkyl ethers.

17. Composition according to any one of Claims 7 to 16, characterized in that the physiologically acceptable medium is thickened by means of thickening and/or gelling agents and contains preservatives, stabilizers, pH regulators, osmotic pressure modifiers, emulsifiers, UVA and UVB filters and antioxidants.

18. Composition according to any one of Claims 7 to 17, characterized in that it also contains surface-active agents chosen from nonionic and amphoteric surface-active agents.

19. Composition according to any one of Claims 7 to 18, for its application as a medication intended to be employed in the therapeutic treatment of hair loss.

20. Process for cosmetic treatment of hair or of the scalp, characterized in that the composition such as defined in any one of Claims 7 to 18 is applied.

21. Use of the composition such as defined in any one of Claims 7 to 18 for the preparation of a medication intended to be employed in the therapeutic treatment of hair loss.

22. compound 2,4-diamino-6-(2-trifluoroethyloxy)pyrimidine 3-oxide

## Claims (Claims for the following Contracting State(s): ES)

1. Process for the preparation of a compound corresponding to the formula: 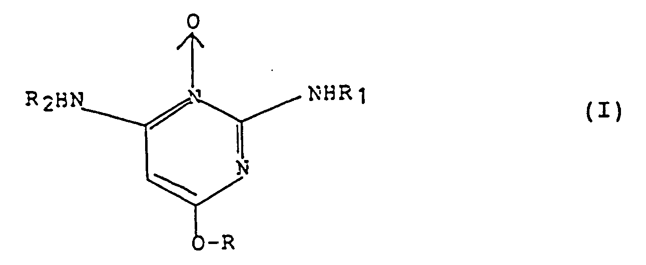 in which:
R₁ and R₂ denote, independently of each other, a hydrogen atom or a carbamoyl group of formula: 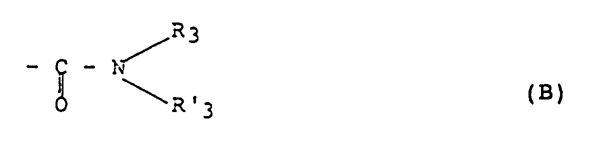 with R'₃ = H or R₃
or an acyl group of formula: 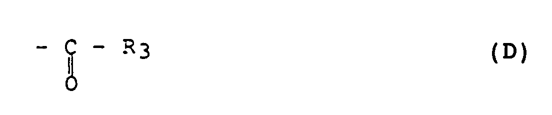 in which R₃ denotes a linear or branched C₁-C₁₈ alkyl radical, a C₂-C₁₈ alkenyl group or a C₅-C₈ cycloalkyl group; R₃ may also denote an aryl or aralkyl radical corresponding to the formula: 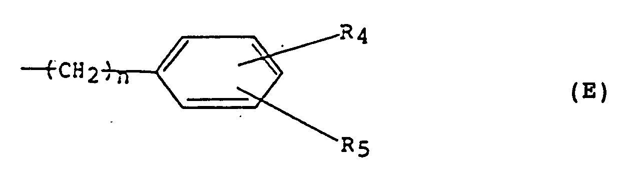 in which:
n is between 0 and 4;
R₄ and/or R₅, independently of each other, denote hydrogen, a lower C₁-C₆ alkyl group, a hydroxyl or C₁-C₆ alkoxy group, a halogen atom or CF₃ group;
R denotes a linear or branched C₁-C₁₈ alkyl or C₂-C₁₈ alkenyl radical, a C₄-C₆ cycloalkyl radical capable of bearing an unsaturation or a C₁-C₆ alkyl radical bearing a phenyl or pyridine group, characterized in that:
(i) when R₁ and R₂ denote hydrogen, the starting material is 2,4-diamino-6-(2,4-dichlorophenoxy)pyrimidine 3-oxide or 2,4-diamino-6-chloropyrimidine 3-oxide, which is reacted with an alcoholate of formula RO^{⊖}Y^{⊕} where R has the specification indicated above and Y is an alkali metal cation such as sodium, potassium or lithium;
(ii) when R₁ and R₂ are carbamoyl or acyl groups, they are obtained from the corresponding 2,4-diamino-6-alkoxypyrimidine 3-oxide derivatives:
- the formation of derivatives in which R₁ and R₂ are carbamoyl groups is obtained by reacting a carbamoyl chloride with the corresponding 2,4-diamino-6-alkoxypyrimidine 3-oxide derivative in a polar solvent, at a temperature between 0 and 100°C;
- the formation of derivatives in which R₁ and R₂ are acyl groups is obtained by reacting an acid chloride or an anhydride with the corresponding 2,4-diamino-6-alkoxypyrimidine 3-oxide derivative in an aprotic polar solvent in the presence of a tertiary amine, at a temperature of between 0 and 50°C.

2. Process according to Claim 1, characterized in that the radical R is chosen from methyl, ethyl, butyl, isobutyl, n-hexyl, n-octyl, n-decyl, lauryl, 2-ethylhexyl, 5-n-hexenyl, 10-undecenyl, cyclohexyl, phenethyl or benzyl groups.

3. Process according to Claim 1 or 2, characterized in that it applies to 2,4-diamino-6-(n-butyloxy)pyrimidine 3-oxide and its cosmetically or pharmaceutically acceptable salts.

4. Process according to Claim 1 or 2, characterized in that it applies to 2,4-diamino-6-ethyloxypyrimidine 3-oxide or its cosmetically or pharmaceutically acceptable salts.

5. Process according to Claim 1, characterized in that the compound belongs to the group of 2,4-diaminopyrimidine 3-oxides, chosen from 2,4-diamino-6-methoxypyrimidine 3-oxide, 2,4-diamino-6-n-hexyloxypyrimidine 3-oxide, 2,4-diamino-6-n-octyloxypyrimidine 3-oxide, 2,4-diamino-6-n-dodecyloxypyrimidine 3-oxide, 2,4-diamino-6-(2-ethylhexyloxy)pyrimidine 3-oxide, 2,4-diamino-6-(5-n-hexenyloxy)pyrimidine 3-oxide, 2,4-diamino-6-(10-undecenyloxy)pyrimidine 3-oxide, 2,4-diamino-6-(2-phenylethyloxy)pyrimidine 3-oxide, or cosmetically or pharmaceutically acceptable salts.

6. Process according to Claim 1, characterized in that the compound prepared is chosen from 2-amino-4-acetamido-6-butyloxypyrimidine 3-oxide, 2,4-diacetamido-6-butyloxypyrimidine 3-oxide and N-(2-amino-6-butyloxy-4-pyrimidinyl)-N'-dimethylurea 3-oxide and its cosmetically or pharmaceutically acceptable salts.

7. Composition intended to be employed in topical application, characterized in that it contains, in a physiologically acceptable medium, at least one compound such as defined in any one of Claims 1 to 6.

8. Composition according to Claim 7, characterized in that it is in the form of an ointment, tincture, cream, pomade, powder, patch, impregnated pad, solution, emulsion, vesicular emulsion, lotion, gel, spray or anhydrous or aqueous suspension, with a view to its pharmaceutical application, and in that it contains at least one compound such as defined in any one of Claims 1 to 6.

9. Composition according to Claim 7 or 8, characterized in that the compounds of formula (I) are present in concentrations of between 0.1 and 10 % by weight relative to the total weight of the composition, and in particular between 0.2 and 5 % by weight.

10. Composition intended to be employed in cosmetics, such as defined in Claim 7, characterized in that it is in the form of a lotion, gel, soap, shampoo, aerosol or foam and that it contains, in a cosmetically acceptable carrier, at least one compound such as defined in any one of Claims 1 to 6, in a concentration of between 0.01 and 7.5 % by weight.

11. Composition according to any one of Claims 7 to 10, characterized in that, in addition to hydrating agents, it contains antiseborrhoeic agents.

12. Composition according to any one of Claims 7 to 11, characterized in that it also contains agents which further improve the activity of the compounds of formula (I) relating to the fresh growth and/or delaying the loss of hair.

13. Composition according to Claim 12, characterized in that it contains, by way of agents which further improve the activity of the fresh growth and/or delaying the loss of hair, nicotinic acid esters, steroid or nonsteroid antiinflammatory agents, retinoids, antibacterial agents, calcium antagonist agents, hormones, antiandrogen agents or scavengers for OH radicals.

14. Composition according to Claim 12, characterized in that it contains, by way of compounds further improving the activity relating to the fresh growth and/or delaying the loss of hair, compounds chosen from diazoxide, spiroxazone, phospholipids, linolenic and linoleic acids, salicylic acid and its derivatives, hydrocarboxylic or ketocarboxylic acids, their esters, lactones and their corresponding salts, anthralin or 1,8,9-trihydroxyanthracene, carotenoids, 5,8,11,14-eicosatetraynoic and 5,8,11-eicosatriynoic acids and their esters and amides.

15. Composition according to any one of Claims 7 to 14, characterized in that the physiologically acceptable medium consists of water, a mixture of water and of one or more organic solvent(s) or of a mixture of organic solvents, the organic solvents being pharmaceutically or cosmetically acceptable.

16. Composition according to Claim 15, characterized in that the solvents are chosen from lower C₁-C₄ alcohols, alkylene glycols and mono- and dialkylene glycol alkyl ethers.

17. Composition according to any one of Claims 7 to 16, characterized in that the physiologically acceptable medium is thickened by means of thickening and/or gelling agents and contains preservatives, stabilizers, pH regulators, osmotic pressure modifiers, emulsifiers, UVA and UVB filters and antioxidants.

18. Composition according to any one of Claims 7 to 17, characterized in that it also contains surface-active agents chosen from nonionic and amphoteric surface-active agents.

19. Composition according to any one of Claims 7 to 18, for its application as a medication intended to be employed in the therapeutic treatment of hair loss.

20. Process for the preparation of a composition intended to be employed in topical application, characterized in that there is introduced into a physiologically acceptable medium, in proportions of 0.1 to 10 % by weight relative to the total weight of the composition, at least one compound of formula (I): 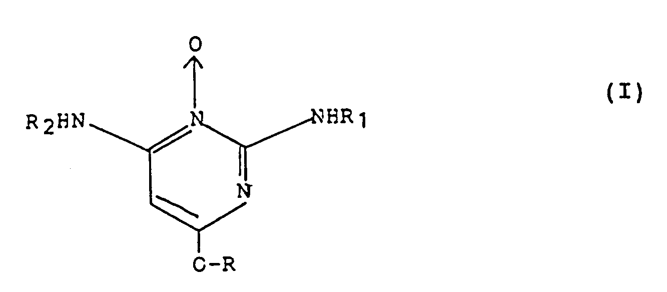 in which:
R₁ and R₂ denote, independently of each other, a hydrogen atom or a carbamoyl group of formula: 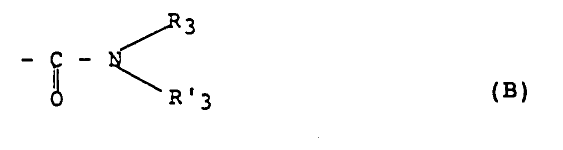 with R'₃ = H or R₃
or an acyl group of formula: 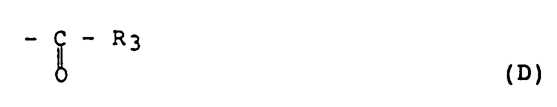 in which R₃ denotes a linear or branched C₁-C₁₈ alkyl radical, a C₂-C₁₈ alkenyl group or a C₅-C₈ cycloalkyl group; R₃ may also denote an aryl or aralkyl radical corresponding to the formula: 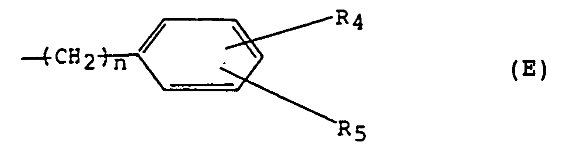 in which:
n is between 0 and 4;
R₄ and/or R₅, independently of each other, denote hydrogen, a lower C₁-C₆ alkyl group, a hydroxyl or C₁-C₆ alkoxy group, a halogen atom or a CF₃ group;
R denotes a linear or branched C₁-C₁₈ alkyl or C₂-C₁₈ alkenyl radical, a C₄-C₆ cycloalkyl radical capable of bearing an unsaturation or a C₁-C₆ alkyl radical bearing a phenyl or pyridine group.

21. compound 2,4-diamino-6-(2-trifluoroethyloxy)pyrimidine 3-oxide.

## Patentansprüche (Patentansprüche für folgende(n) Vertragsstaat(en): AT, BE, CH, DE, FR, GB, IT, LI, NL, SE)

1. Verbindung, dadurch **gekennzeichnet**, daß sie die Formel aufweist: 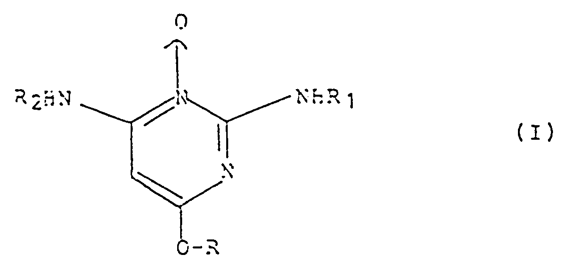 worin gilt:
R₁ und R₂ bedeuten, unabhängig voneinander, ein Wasserstoffatom, eine Carbamoylgruppe der Formel: 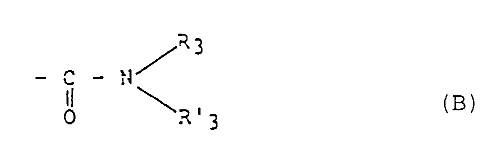 mit R'₃ = H oder R₃
oder eine Acylgruppe der Formel: 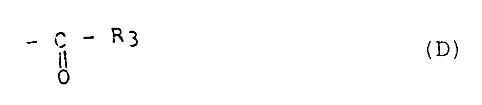 in der R₃ einen linearen oder verzweigten C₁₋₁₈-Alkylrest, eine C₂₋₁₈-Alkenylgruppe, eine C₅₋₈-Cycloalkylgruppe bedeutet oder R₃ auch einen Aryl- oder Aralkylrest der Formel bedeuten kann: 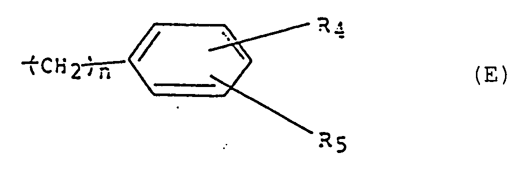 worin gilt:
n ist eine ganze Zahl von 0 bis 4;
R₄ und/oder R₅ bedeuten, unabhängig voneinander, Wasserstoff, eine C₁₋₆-Alkylgruppe, Hydroxylgruppe, C₁₋₆-Alkoxygruppe, ein Halogenatom oder die Gruppe CF₃;
R bedeutet einen linearen oder verzweigten C₁₋₁₈-Alkylrest, C₂₋₁₈-Alkenylrest, einen gegebenenfalls ungesättigten C₄₋₆-Cycloalkylrest oder einen C₁₋₆-Alkylrest, der eine Phenyl- oder Pyridingruppe aufweist.

2. Verbindung gemäß Anspruch 1,
dadurch **gekennzeichnet**, daß
der Rest R aus Methyl-, Ethyl-, Butyl-, Isobutyl-, n-Hexyl-, n-Octyl-, n-Decyl-, Lauryl-, 2-Ethylhexyl-, n-Hex-5-enyl-, Undec-10-enyl-, Cyclohexyl-, Phenethyl- oder Benzylgruppen ausgewählt ist.

3. Verbindung gemäß Anspruch 1 oder 2,
dadurch **gekennzeichnet**, daß
es sich um 2,4-Diamino-6-n-butyloxypyrimidin-3-oxid oder seine kosmetisch oder pharmazeutisch zulässigen Salze handelt.

4. Verbindung gemäß Anspruch 1 oder 2,
dadurch **gekennzeichnet**, daß
es sich um 2,4-Diamino-6-ethyloxypyrimidin-3-oxid oder seine kosmetisch oder pharmazeutisch zulässigen Salze handelt.

5. Verbindung der Familie der 2,4-Diaminopyrimidin-3-oxide gemäß Anspruch 1, ausgewählt 2,4-Diamino-6-methoxypyrimidin-3-oxid, 2,4-Diamino-6-n-hexyloxypyrimidin-3-oxid, 2,4-Diamino-6-n-octyloxypyrimidin-3-oxid, 2,4-Diamino-6-n-dodecyloxypyrimidin-3-oxid, 2,4-Diamino-6(2-ethylhexyloxy)pyrimidin-3-oxid, 2,4-Diamino-6-n-hex-5-enyloxypyrimidin-3-oxid, 2,4-Diamino-6-undec-10-enyloxypyrimidin-3-oxid, 2,4-Diamino-6(2-phenylethyloxy)pyrimidin-3-oxid oder aus den kosmetisch oder pharmazeutisch zulässigen Salzen.

6. Verbindung gemäß Anspruch 1,
dadurch **gekennzeichnet**, daß
sie aus 2-Amino-4-acetamido-6-butyloxypyrimidin-3-oxid, 2,4-Diacetamido-6-butyloxypyrimidin-3-oxid, N-(2-Amino-3-oxid-6-butyloxypyrimidinyl-4),N'-dimethylharnstoff und aus deren kosmetisch oder pharmazeutisch zulässigen Salzen ausgewählt ist.

7. Zusammensetzung zur Verwendung in topischer Applikation, dadurch **gekennzeichnet**, daß
sie, in einem physiologisch zulässigen Milieu, mindestens eine in jedem der Ansprüche 1 bis 6 definierte Verbindung enthält.

8. Zusammensetzung gemäß Anspruch 7,
dadurch **gekennzeichnet**, daß
sie in Form einer Salbe, Tinktur, Creme, Pomade, eines Pulvers, Tupfers, getränkten Tampon, einer Lösung, Emulsion, bläschenartigen Emulsion, Lotion, eines Gels, Spray oder einer wasserfreien oder wässrigen Suspension vorliegt, und zwar im Hinblick auf ihre pharmazeutische Applikation, und daß sie mindestens eine in jedem der Ansprüche 1 bis 6 definierte Verbindung enthält.

9. Zusammensetzung gemäß Anspruch 7 oder 8,
dadurch **gekennzeichnet**, daß
die Verbindungen der Formel (I) in Konzentrationen von 0,1 bis 10, insbesondere von 0,2 bis 5, Gew.% vorhanden sind, bezogen auf das Gesamtgewicht der Zusammensetzung.

10. Zusammensetzung gemäß Anspruch 7 zur kosmetischen Verwendung,
dadurch **gekennzeichnet**, daß
sie in Form einer Lotion, eines Gels, einer Seife, eines Schampoo, Aerosols oder Schaums vorliegt und, in einem auf dem kosmetischen Plan zulässigen Trägermittel, mindestens eine in jedem der Ansprüche 1 bis 6 definierte Verbindung in einer Konzentration von 0,1 bis 7,5 Gew.% enthält.

11. Zusammensetzung gemäß jedem der Ansprüche 7 bis 10, dadurch **gekennzeichnet**, daß
sie, zusätzlich zu hydratisierenden Mitteln, antiseborrheische Mittel enthält.

12. Zusammensetzung gemäß jedem der Ansprüche 7 bis 11, dadurch **gekennzeichnet**, daß
sie auch Mittel enthält, die die Wirksamkeit der Verbindungen der Formel (I) auf der Ebene des Wachstumsanstoßes für die Haare und/oder des Zurückdrängens von deren Ausfall noch verbessern.

13. Zusammensetzung gemäß Anspruch 12,
dadurch **gekennzeichnet**, daß
sie als Mittel, die die Wirksamkeit des Wachstumsanstoßes für die Haare und/oder des Zurückdrängens von deren Ausfall noch verstärken, Nikotinsäureester, steroide oder nicht-steroide entzündungshemmende Mittel, Retinoide, anitbakterielle Mittel, antagonistische Mittel des Calciums, Hormone, antiandrogene Mittel, Anfangmittel für OH-Radikale enthält.

14. Zusammensetzung gemäß Anspruch 12,
dadurch **gekennzeichnet**, daß
sie als Verbindungen, die die Wirksamkeit auf den Wachstumsanstoß der Haare und/oder das Zurückdrängen von deren Ausfall noch verstärken, Verbindungen enthält, ausgewählt aus Diazoxid, Spiroxazon, Phospholipiden, Linol- und Linolensäure, Salicylsäure und ihren Derivaten, Hydroxycarboxyl- oder Ketocarboxylsäuren, ihren Estern, Lactonen und ihren entsprechenden Salzen, Anthralin oder 1,8,9-Trihydroxyanthracen, Carotenoiden, Eicosa-5,8,11,14-tetrain-, Eicosa-5,8,11-triinsäure, deren Estern und Amiden.

15. Zusammensetzung gemäß jedem der Ansprüche 7 bis 14, dadurch **gekennzeichnet**, daß
das physiologisch verträgliche Milieu aus Wasser, einer Mischung aus Wasser oder einem oder mehreren organischen Lösungsmitteln oder einer Mischung aus organischen Lösungsmitteln zusammengesetzt ist, wobei die organischen Lösungsmittel pharmazeutisch oder kosmetisch zulässig sind.

16. Zusammensetzung gemäß Anspruch 15,
dadurch **gekennzeichnet**, daß
die Lösungsmittel aus C₁₋₄-Niedrigalkoholen, Alkylenglycolen, Alkylethern von Mono- und Dialkylenglycol ausgewählt sind.

17. Zusammensetzung gemäß einem jeden der Ansprüche 7 bis 16,
dadurch **gekennzeichnet**, daß
das physiologisch zulässige Milieu mit Verdickungs- und/oder Geliermitteln verdickt ist und Konservierungsmittel, Stabilisiermittel, pH-Reguliermittel, Modifiziermittel des osmotischen Drucks, Emulgatoren, Filterstoffe für UV-A und UV-B, Antioxidantien enthält.

18. Zusammensetzung gemäß einem jeden der Ansprüche 7 bis 17,
dadurch **gekennzeichnet**, daß
sie auch oberflächenaktive Mittel enthält, ausgewählt aus nicht-ionischen und amphoteren oberflächenaktiven Mitteln.

19. Zusammensetzung gemäß einem jeden der Ansprüche 7 bis 18 zur Verwendung als Medikament zur therapeutischen Behandlung des Ausfalls von Haaren.

20. Verfahren zur kosmetischen Behandlung der Haare oder behaarter Haut,
dadurch **gekennzeichnet**, daß
man die in jedem der Ansprüche 7 bis 18 definierte Zusammensetzung zur Anwendung bringt.

21. Verwendung der in jedem der Ansprüche 7 bis 18 definierten Zusammensetzung zur Herstellung eines Medikaments zur Verwendung bei der therapeutischen Behandlung von Haarausfall.

22. Die Verbindung 2,4-Diamino-6-(2-trifluorethyloxy)pyrimidin-3-oxid.

## Patentansprüche (Patentansprüche für folgende(n) Vertragsstaat(en): ES)

1. Verfahren zur Herstellung einer Verbindung der Formel: 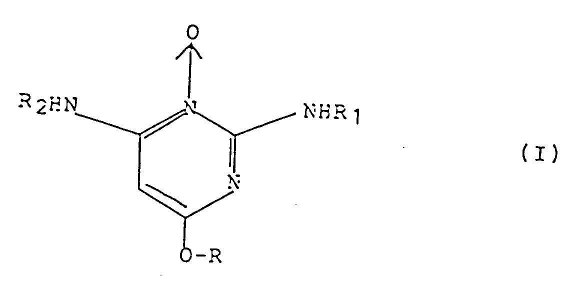 worin gilt:
R₁ und R₂ bedeuten, unabhängig voneinander, ein Wasserstoffatom, eine Carbamoylgruppe der Formel: 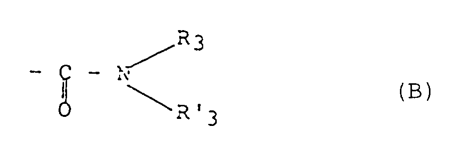 mit R'₃ = H oder R₃
oder eine Acylgruppe der Formel: 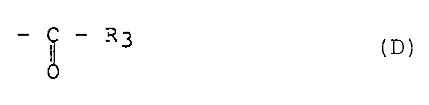 in der R₃ einen linearen oder verzweigten C₁₋₁₈-Alkylrest, eine C₂₋₁₈-Alkenylgruppe, eine C₅₋₈-Cycloalkylgruppe bedeutet oder R₃ auch einen Aryl- oder Aralkylrest der Formel bedeuten kann: 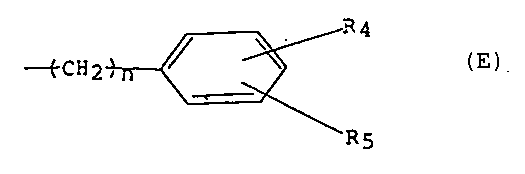 worin gilt:
n ist eine ganze Zahl von 0 bis 4;
R₄ und/oder R₅ bedeuten, unabhängig voneinander, Wasserstoff, eine C₁₋₆-Alkylgruppe, Hydroxylgruppe, C₁₋₆-Alkoxygruppe, ein Halogenatom oder die Gruppe CF₃;
R bedeutet einen linearen oder verzweigten C₁₋₁₈-Alkylrest, C₂₋₁₈-Alkenylrest, einen gegebenenfalls ungesättigten C₄₋₆-Cycloalkylrest oder einen C₁₋₆-Alkylrest, der eine Phenyl- oder Pyridingruppe aufweist,
dadurch **gekennzeichnet**, daß man:
(i) wenn R₁ und R₂ Wasserstoff bedeuten, von 2,4-Diamino-6-(2,4-dichlorphenoxy)pyrimidin-3-oxid oder von 2,4-Diamino-6-chlorpyrimidin-3-oxid ausgeht, die man mit einem Alkoholat der Formel RO⁻Y⁺ reagieren läßt, worin R die oben angegebene Bedeutung hat und Y ein Alkalikation wie Natrium, Kalium, Lithium ist;
(ii) wenn R₁ und R₂ Carbamoyl- oder Acylgruppen sind, die Verbindungen aus den entsprechenden Derivaten von 2,4-Diamino-6-alkoxypyrimidin-3-oxid erhält, wobei
- der Erhalt der Derivate, für die R₁ und R₂ Carbamoylgruppen sind, bewerkstelligt wird, indem man ein Carbamoylchlorid mit dem entsprechenden 2,4-Diamino-6-alkoxypyrimidin-3-oxid-Derivat in einem polaren Lösungsmittel bei einer Temperatur von 0 bis 100°C reagieren läßt, und
- der Erhalt von Derivaten, für die R₁ und R₂ Acylgruppen sind, bewerkstelligt wird, indem man ein Säurechlorid oder
- anhydrid mit dem entsprechenden 2,4-Diamino-6-alkoxypyrimidin-3-oxid-Derivat in einem polaren aprotischen Lösungsmittel, in Gegenwart eines tertiären Amins bei einer Temperatur von 0 bis 50°C reagieren läßt.

2. Verfahren gemäß Anspruch 1,
dadurch **gekennzeichnet**, daß
der Rest R aus Methyl-, Ethyl-, Butyl-, Isobutyl-, n-Hexyl-, n-Octyl-, n-Decyl-, Lauryl-, 2-Ethylhexyl-, n-Hex-5-enyl-, Undec-10-enyl-, Cyclohexyl-, Phenethyl- oder Benzylgruppen ausgewählt ist.

3. Verfahren gemäß Anspruch 1 oder 2,
dadurch **gekennzeichnet**, daß
es sich um 2,4-Diamino-6-n-butyloxypyrimidin-3-oxid oder seine kosmetisch oder pharmazeutisch zulässigen Salze handelt.

4. Verfahren gemäß Anspruch 1 oder 2,
dadurch **gekennzeichnet**, daß
es sich um 2,4-Diamino-6-ethyloxypyrimidin-3-oxid oder seine kosmetisch oder pharmazeutisch zulässigen Salze handelt.

5. Verfahren gemäß Anspruch 1,
dadurch **gekennzeichnet**, daß
die Verbindung zur Familie der 2,4-Diaminopyrimidin-3-oxide gehört, ausgewählt aus 2,4-Diamino-6-methoxypyrimidin-3-oxid, 2,4-Diamino-6-n-hexyloxypyrimidin-3-oxid, 2,4-Diamino-6-n-octyloxypyrimidin-3-oxid, 2,4-Diamino-6-n-dodecyloxypyrimidin-3-oxid, 2,4-Diamino-6(2-ethylhexyloxy)pyrimidin-3-oxid, 2,4-Diamino-6-n-hex-5-enyloxypyrimidin-3-oxid, 2,4-Diamino-6-undec-10-enyloxypyrimidin-3-oxid, 2,4-Diamino-6(2-phenylethyloxy)pyrimidin-3-oxid oder aus den kosmetisch oder pharmazeutisch zulässigen Salzen.

6. Verfahren gemäß Anspruch 1,
dadurch **gekennzeichnet**, daß
die hergestellte Verbindung aus 2-Amino-4-acetamido-6-butyloxypyrimidin-3-oxid, 2,4-Diacetamido-6-butyloxypyrimidin-3-oxid, N-(2-Amino-3-oxid-6-butyloxypyrimidinyl-4),N'-dimethylharnstoff und aus deren kosmetisch oder pharmazeutisch zulässigen Salzen ausgewählt ist.

7. Zusammensetzung zur Verwendung in topischer Applikation, dadurch **gekennzeichnet**, daß
sie, in einem physiologisch zulässigen Milieu, mindestens eine in jedem der Ansprüche 1 bis 6 definierte Verbindung enthält.

8. Zusammensetzung gemäß Anspruch 7,
dadurch **gekennzeichnet**, daß
sie in Form einer Salbe, Tinktur, Creme, Pomade, eines Pulvers, Tupfers, getränkten Tampon, einer Lösung, Emulsion, bläschenartigen Emulsion, Lotion, eines Gels, Spray oder einer wasserfreien oder wässrigen Suspension vorliegt, und zwar im Hinblick auf ihre pharmazeutische Applikation, und daß sie mindestens eine in jedem der Ansprüche 1 bis 6 definierte Verbindung enthält.

9. Zusammensetzung gemäß Anspruch 7 oder 8,
dadurch **gekennzeichnet**, daß
die Verbindungen der Formel (I) in Konzentrationen von 0,1 bis 10, insbesondere von 0,2 bis 5, Gew.% vorhanden sind, bezogen auf das Gesamtgewicht der Zusammensetzung.

10. Zusammensetzung gemäß Anspruch 7 zur kosmetischen Verwendung,
dadurch **gekennzeichnet**, daß
sie in Form einer Lotion, eines Gels, einer Seife, eines Schampoo, Aerosols oder Schaums vorliegt und, in einem auf dem kosmetischen Plan zulässigen Trägermittel, mindestens eine in jedem der Ansprüche 1 bis 6 definierte Verbindung in einer Konzentration von 0,1 bis 7,5 Gew.% enthält.

11. Zusammensetzung gemäß jedem der Ansprüche 7 bis 10, dadurch **gekennzeichnet**, daß
sie, zusätzlich zu hydratisierenden Mitteln, antiseborrheische Mittel enthält.

12. Zusammensetzung gemäß jedem der Ansprüche 7 bis 11, dadurch **gekennzeichnet**, daß
sie auch Mittel enthält, die die Wirksamkeit der Verbindungen der Formel (I) auf der Ebene des Wachstumsanstoßes für die Haare und/oder des Zurückdrängens von deren Ausfall noch verbessern.

13. Zusammensetzung gemäß Anspruch 12,
dadurch **gekennzeichnet**, daß
sie als Mittel, die die Wirksamkeit des Wachstumsanstoßes für die Haare und/oder des Zurückdrängens von deren Ausfall noch verstärken, Nikotinsäureester, steroide oder nicht-steroide entzündungshemmende Mittel, Retinoide, antibakterielle Mittel, antagonistische Mittel des Calciums, Hormone, antiandrogene Mittel, Abfangmittel für OH-Radikale enthält.

14. Zusammensetzung gemäß Anspruch 12,
dadurch **gekennzeichnet**, daß
sie als Verbindungen, die die Wirksamkeit auf den Wachstumsanstoß der Haare und/oder das Zurückdrängen von deren Ausfall noch verstärken, Verbindungen enthält, ausgewählt aus Diazoxid, Spiroxazon, Phospholipiden, Linol- und Linolensäure, Salicylsäure und ihren Derivaten, Hydroxycarboxyl- oder Ketocarboxylsäuren, ihren Estern, Lactonen und ihren entsprechenden Salzen, Anthralin oder 1,8,9-Trihydroxyanthracen, Carotenoiden, Eicosa-5,8,11,14-tetrain-, Eicosa-5,8,11-triinsäure, deren Estern und Amiden.

15. Zusammensetzung gemäß jedem der Ansprüche 7 bis 14, dadurch **gekennzeichnet**, daß
das physiologisch verträgliche Milieu aus Wasser, einer Mischung aus Wasser oder einem oder mehreren organischen Lösungsmitteln oder einer Mischung aus organischen Lösungsmitteln zusammengesetzt ist, wobei die organischen Lösungsmittel pharmazeutisch oder kosmetisch zulässig sind.

16. Zusammensetzung gemäß Anspruch 15,
dadurch **gekennzeichnet**, daß
die Lösungsmittel aus C₁₋₄-Niedrigalkoholen, Alkylenglycolen, Alkylethern von Mono- und Dialkylenglycol ausgewählt sind.

17. Zusammensetzung gemäß einem jeden der Ansprüche 7 bis 16,
dadurch **gekennzeichnet**, daß
das physiologisch zulässige Milieu mit Verdickungs- und/oder Geliermitteln verdickt ist und Konservierungsmittel, Stabilisiermittel, pH-Reguliermittel, Modifiziermittel des osmotischen Drucks, Emulgatoren, Filterstoffe für UV-A und UV-B, Antioxidantien enthält.

18. Zusammensetzung gemäß einem jeden der Ansprüche 7 bis 17,
dadurch **gekennzeichnet**, daß
sie auch oberflächenaktive Mittel enthält, ausgewählt aus nicht-ionischen und amphoteren oberflächenaktiven Mitteln.

19. Zusammensetzung gemäß einem jeden der Ansprüche 7 bis 18 zur Verwendung als Medikament zur therapeutischen Behandlung des Ausfalls von Haaren.

20. Verfahren zur Herstellung einer Zusammensetzung zur Verwendung in topischer Applikation,
dadurch **gekennzeichnet**, daß
man in ein physiologisch zulässiges Milieu in Mengenanteilen von 0,1 bis 10 Gew.%, bezogen auf das Gesamtgewicht der Zusammensetzung, mindestens eine Verbindung der Formel (I) einbringt: 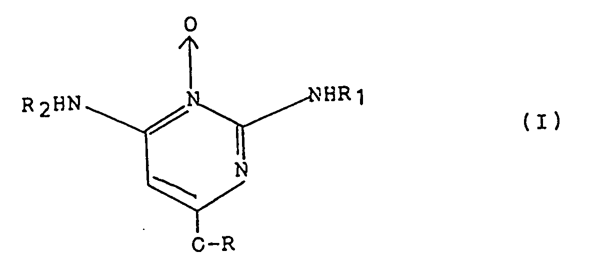 worin gilt:
R₁ und R₂ bedeuten, unabhängig voneinander, ein Wasserstoffatom, eine Carbamoylgruppe der Formel: 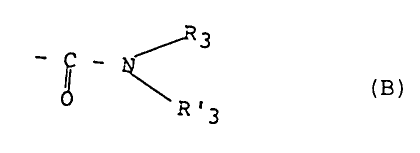 mit R'₃ = H oder R₃
oder eine Acylgruppe der Formel: 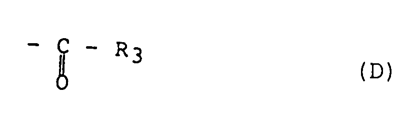 in der R₃ einen linearen oder verzweigten C₁₋₁₈-Alkylrest, eine C₂₋₁₈-Alkenylgruppe, eine C₅₋₈-Cycloalkylgruppe bedeutet oder R₃ auch einen Aryl- oder Aralkylrest der Formel bedeuten kann: 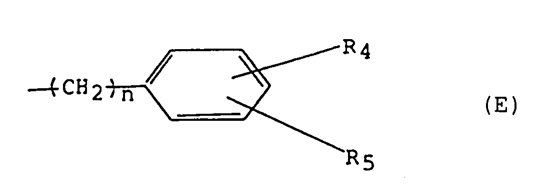 worin gilt:
n ist eine ganze Zahl von 0 bis 4;
R₄ und/oder R₅ bedeuten, unabhängig voneinander, Wasserstoff, eine C₁₋₆-Alkylgruppe, Hydroxylgruppe, C₁₋₆-Alkoxygruppe, ein Halogenatom oder die Gruppe CF₃;
R bedeutet einen linearen oder verzweigten C₁₋₁₈-Alkylrest, C₂₋₁₈-Alkenylrest, einen gegebenenfalls ungesättigten C₄₋₆-Cycloalkylrest oder einen C₁₋₆-Alkylrest, der eine Phenyl- oder Pyridingruppe aufweist.

21. Die Verbindung 2,4-Diamino-6-(2-trifluorethyloxy)pyrimidin-3-oxid.
